# EUROPEAN PATENT APPLICATION

(11) **EP 4 349 836 A1**
(43) Date of publication of application: **10.04.2024**
(21) Application number: 22810541.7
(22) Date of filing: 24.05.2022
(51) Int. Cl.: C07D 471/04, C07D 401/12, A61K 31/4375, A61P 35/00

(54) **NITROGEN-CONTAINING HETEROCYCLIC COMPOUND, PREPARATION METHOD THEREFOR, AND APPLICATION THEREOF IN MEDICINES**

(30) Priority: 24.05.2021 CN 202110565410; 22.06.2021 CN 202110694022; 28.07.2021 CN 202110856289; 02.03.2022 CN 202210198679
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); DONG, Huaide, Shanghai 200245 (CN); CAI, Guodong, Shanghai 200245 (CN); HE, Feng, Shanghai 200245 (CN); TAO, Weikang, Shanghai 200245 (CN)
(74) Representative: Potter Clarkson
(86) International application number: PCT/CN2022/094612
(87) International publication number: WO 2022/247816

(57) **Abstract**

A nitrogen-containing heterocyclic compound, a preparation method therefor, and an application thereof in medicines. Specifically, the present disclosure relates to a nitrogen-containing heterocyclic compound as represented by general formula (IM), a preparation method therefor, a pharmaceutical composition comprising the compound, a use of the pharmaceutical composition serving as a therapeutic agent, in particular as a PARP1 inhibitor, and a use of the pharmaceutical composition in the preparation of a drug for treating and/or preventing cancer.

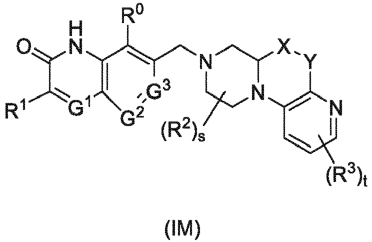

## Description

### TECHNICAL FIELD

The present disclosure belongs to the field of pharmaceutics, and relates to a nitrogen-containing heterocyclic compound, a preparation method therefor, and pharmaceutical use thereof. In particular, the present disclosure relates to a nitrogen-containing heterocyclic compound of general formula (IM), a preparation method therefor, a pharmaceutical composition comprising the compound, use thereof as a PARP1 inhibitor, and use thereof in the preparation of a drug for treating and/or preventing cancer.

### BACKGROUND

Poly(ADP-ribose) polymerase 1 (PARP1) was first reported over 50 years ago, and was gradually found to play an important role in DNA repair, maintenance of genomic integrity, regulation of various metabolic and signal transduction processes, and the like. PARP1 is capable of catalyzing the transfer of ADP-ribose residues from NAD+ to a target substrate, thereby constructing a poly(ADP-ribose) (PAR) chain. The formation and clearance of the PAR chain occur in almost all eukaryotic cells.

ADP-ribosylation is a post-translational modification of proteins that is widely present in various physiological and pathological processes, and refers to the binding of one or more ADP-ribose units to specific sites of proteins by catalysis with enzymes. PARP1 is the first member of the PARP superfamily, which consists of proteins having homology to PARP1. There are 17 members, of which 4 (PARP1, PARP2, PARP5A, and PARP5B) are capable of synthesizing the PAR chain. Most other enzymes in the family can only construct a single ADP-ribose unit and are therefore classified as mono(ADP-ribosyl)ases (MARs).

PARP1 and PARP2 have been extensively studied for their roles in DNA damage repair. PARP1 is activated by DNA damage and functions to catalyze the formation of a poly(ADP-ribose) (PAR) chain on a target protein. This post-translational modification, referred to as poly-ADP-ribosylation (PARylation), can mediate the recruitment of other DNA repair factors to the DNA damage. Upon completion of this recruitment task, PARP auto-PARylation triggers the release of bound PARP from DNA, thereby allowing access to other DNA repair proteins to complete the repair. Therefore, the binding of PARP to damaged sites, its catalytic activity, and final release from DNA are all important steps in the response of cancer cells to DNA damage caused by chemotherapeutic agents and radiotherapy.

The inhibition of PARP family enzymes has been used as a strategy to selectively kill cancer cells by inactivating complementary DNA repair pathways. Numerous preclinical and clinical studies have shown that tumor cells with harmful changes of the key tumor suppressor protein BRCA1 or BRCA2 involved in the double-strand DNA break (DSB) repair through harmful recombination (HR) are selectively sensitive to small molecules. DNA repair enzymes and PARP family inhibitors. The homologous recombination repair (HRR) pathway of such tumors is deficient and depends on the survival function of PARP enzymes. Although PARP inhibitor therapy is mainly directed against BRCA-mutated cancers, PARP inhibitors have been clinically tested in non-BRCA-mutated tumors that exhibit homologous recombination deficiency (HRD). PARP inhibitors with increased selectivity for PARP1 may have improved efficacy and reduced toxicity compared to other PARP1/2 inhibitors. We believe that a selective and strong inhibition of PARP1 will lead to the capture of PARP1 on DNA, resulting in DNA double-strand breaks (DSBs) by the collapse of replication forks in the S phase. PARP1-DNA capture is an effective mechanism to selectively kill tumor cells with HRD.

Therefore, there is an urgent clinical need for effective and safe PARP inhibitors, particularly a PARP inhibitor with the selectivity for PARP1.

Related patent applications that have been disclosed include WO2021013735A1, WO202l260092A1, WO2009053373A1, WO2008107478A1, and the like.

### SUMMARY

The present disclosure aims to provide a compound of general formula (IM) or a pharmaceutically acceptable salt thereof: 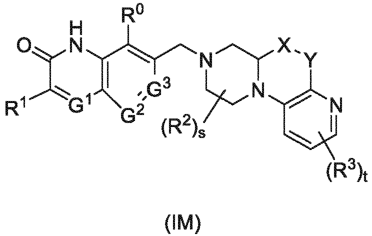 wherein:
X and Y are identical or different and are each independently selected from the group consisting of (CR^{4a}R^{4b})ₘ, NR⁵(CR^{4c}R^{4d})ᵣ, C(O)NR⁵, NR⁵C(O), C(O), and O(CR^{4e}R^{4f})ₙ;
R^{4a}, R^{4b}, R^{4c}, R^{4d}, R^{4e}, and R^{4f} are identical or different and are each independently selected from the group consisting of hydrogen atom, halogen, alkyl, haloalkyl, hydroxyalkyl, cycloalkyl, heterocyclyl, aryl, and heteroaryl;
R⁵ is selected from the group consisting of hydrogen atom, alkyl, haloalkyl, hydroxyalkyl, cycloalkyl, heterocyclyl, aryl, and heteroaryl;
G¹, G², and G³ are identical or different and are each independently CR⁶ or a nitrogen atom;
R⁰, R¹, and R⁶ are identical or different and are each independently selected from the group consisting of hydrogen atom, halogen, alkyl, alkoxy, haloalkyl, haloalkoxy, hydroxyalkyl, cyano, -NR^{7a}R^{7b}, hydroxy, -C(O)R⁸, -C(O)OR⁸, -C(O)NR^{7a}R^{7b}, -S(O)ₚR⁸, cycloalkyl, heterocyclyl, aryl, and heteroaryl, wherein the alkyl, alkoxy, cycloalkyl, heterocyclyl, aryl, and heteroaryl are each independently optionally substituted with one or more substituents selected from the group consisting of halogen, oxo, alkyl, alkoxy, haloalkyl, haloalkoxy, cyano, -NR^{9a}R^{9b}, hydroxy, hydroxyalkyl, cycloalkyl, heterocyclyl, aryl, and heteroaryl;
each R² is identical or different and is independently selected from the group consisting of halogen, alkyl, alkoxy, haloalkyl, haloalkoxy, oxo, cyano, -NR^{7a}R^{7b}, hydroxy, and hydroxyalkyl;
each R³ is identical or different and is independently selected from the group consisting of halogen, alkyl, alkoxy, haloalkyl, haloalkoxy, hydroxyalkyl, cyano, -NR^{7a}R^{7b}, hydroxy, -C(O)R⁸, -C(O)OR⁸, -C(O)NR^{7a}R^{7b}, -S(O)ₚR⁸, cycloalkyl, heterocyclyl, aryl, and heteroaryl, wherein the alkyl, alkoxy, cycloalkyl, heterocyclyl, aryl, and heteroaryl are each independently optionally substituted with one or more substituents selected from the group consisting of halogen, oxo, alkyl, alkoxy, haloalkyl, haloalkoxy, cyano, -NR^{9a}R^{9b}, hydroxy, hydroxyalkyl, cycloalkyl, heterocyclyl, aryl, and heteroaryl;
R^{7a}, R^{7b}, R^{9a}, and R^{9b} are identical or different and are each independently selected from the group consisting of hydrogen atom, alkyl, hydroxyalkyl, cycloalkyl, and heterocyclyl, wherein the alkyl, cycloalkyl, and heterocyclyl are each independently optionally substituted with one or more substituents selected from the group consisting of halogen, alkyl, alkoxy, haloalkyl, and haloalkoxy;
or R^{7a} and R^{7b}, together with the nitrogen atom to which they are attached, form heterocyclyl; R^{9a} and R^{9b}, together with the nitrogen atom to which they are attached, form heterocyclyl; the heterocyclyl formed is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
R⁸ is selected from the group consisting of hydrogen atom, alkyl, hydroxyalkyl, cycloalkyl, and heterocyclyl, wherein the alkyl, cycloalkyl, and heterocyclyl are each independently optionally substituted with one or more substituents selected from the group consisting of halogen, alkyl, alkoxy, haloalkyl, and haloalkoxy;
p is 0, 1, or 2;
m is 0, 1, 2, 3, or 4;
n is 0, 1, 2, 3, or 4;
r is 0, 1, 2, 3, or 4;
s is 0, 1, 2, 3, or 4; and
t is 0, 1, 2, or 3.

In some embodiments of the present disclosure, the compound of general formula (IM) or the pharmaceutically acceptable salt thereof is a compound of general formula (I) or a pharmaceutically acceptable salt thereof: 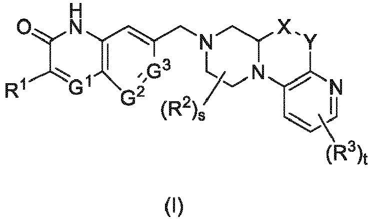 wherein:
X and Y are identical or different and are each independently selected from the group consisting of (CR^{4a}R^{4b})ₘ, NR⁵(CR^{4c}R^{4d})ᵣ, C(O)NR⁵, NR⁵C(O), C(O), and O(CR^{4e}R^{4f})ₙ;
R^{4a}, R^{4b}, R^{4c}, R^{4d}, R^{4e}, and R^{4f} are identical or different and are each independently selected from the group consisting of hydrogen atom, halogen, alkyl, haloalkyl, hydroxyalkyl, cycloalkyl, heterocyclyl, aryl, and heteroaryl;
R⁵ is selected from the group consisting of hydrogen atom, alkyl, haloalkyl, hydroxyalkyl, cycloalkyl, heterocyclyl, aryl, and heteroaryl;
G¹, G², and G³ are identical or different and are each independently CR⁶ or a nitrogen atom;
R¹ and R⁶ are identical or different and are each independently selected from the group consisting of hydrogen atom, halogen, alkyl, alkoxy, haloalkyl, haloalkoxy, hydroxyalkyl, cyano, -NR^{7a}R^{7b}, hydroxy, -C(O)R⁸, -C(O)OR⁸, -C(O)NR^{7a}R^{7b}, -S(O)ₚR⁸, cycloalkyl, heterocyclyl, aryl, and heteroaryl, wherein the alkyl, alkoxy, cycloalkyl, heterocyclyl, aryl, and heteroaryl are each independently optionally substituted with one or more substituents selected from the group consisting of halogen, oxo, alkyl, alkoxy, haloalkyl, haloalkoxy, cyano, -NR^{9a}R^{9b}, hydroxy, hydroxyalkyl, cycloalkyl, heterocyclyl, aryl, and heteroaryl;
each R² is identical or different and is independently selected from the group consisting of halogen, alkyl, alkoxy, haloalkyl, haloalkoxy, oxo, cyano, -NR^{7a}R^{7b}, hydroxy, and hydroxyalkyl;
each R³ is identical or different and is independently selected from the group consisting of halogen, alkyl, alkoxy, haloalkyl, haloalkoxy, hydroxyalkyl, cyano, -NR^{7a}R^{7b}, hydroxy, -C(O)R⁸, -C(O)OR⁸, -C(O)NR^{7a}R^{7b}, -S(O)ₚR⁸, cycloalkyl, heterocyclyl, aryl, and heteroaryl, wherein the alkyl, alkoxy, cycloalkyl, heterocyclyl, aryl, and heteroaryl are each independently optionally substituted with one or more substituents selected from the group consisting of halogen, oxo, alkyl, alkoxy, haloalkyl, haloalkoxy, cyano, -NR^{9a}R^{9b}, hydroxy, hydroxyalkyl, cycloalkyl, heterocyclyl, aryl, and heteroaryl;
R^{7a}, R^{7b}, R^{9a}, and R^{9b} are identical or different and are each independently selected from the group consisting of hydrogen atom, alkyl, hydroxyalkyl, cycloalkyl, and heterocyclyl, wherein the alkyl, cycloalkyl, and heterocyclyl are each independently optionally substituted with one or more substituents selected from the group consisting of halogen, alkyl, alkoxy, haloalkyl, and haloalkoxy;
or R^{7a} and R^{7b}, together with the nitrogen atom to which they are attached, form heterocyclyl; R^{9a} and R^{9b}, together with the nitrogen atom to which they are attached, form heterocyclyl; the heterocyclyl formed is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
R⁸ is selected from the group consisting of hydrogen atom, alkyl, hydroxyalkyl, cycloalkyl, and heterocyclyl, wherein the alkyl, cycloalkyl, and heterocyclyl are each independently optionally substituted with one or more substituents selected from the group consisting of halogen, alkyl, alkoxy, haloalkyl, and haloalkoxy;
p is 0, 1, or 2;
m is 0, 1, 2, 3, or 4;
n is 0, 1, 2, 3, or 4;
r is 0, 1, 2, 3, or 4;
s is 0, 1, 2, 3, or 4; and
t is 0, 1, 2, or 3.

In some embodiments of the present disclosure, the compound of general formula (IM) or general formula (I) or the pharmaceutically acceptable salt thereof is provided, wherein X is (CR^{4a}R^{4b})ₘ or C(O); wherein R^{4a}, R^{4b}, and m are as defined in general formula (IM); preferably, X is selected from the group consisting of CH₂, CH₂CH₂, and C(O).

In some embodiments of the present disclosure, the compound of general formula (IM) or general formula (I) or the pharmaceutically acceptable salt thereof is provided, wherein Y is O(CR^{4e}R^{4f})ₙ or NR⁵(CR^{4c}R^{4d})ᵣ; wherein R^{4c}, R^{4d}, R^{4e}, R^{4f}, R⁵, n, and r are as defined in general formula (IM); preferably, Y is selected from the group consisting of O, OCH₂, NH, and NCH₃.

In some embodiments of the present disclosure, the compound of general formula (IM) or general formula (I) or the pharmaceutically acceptable salt thereof is provided, wherein X is (CR^{4a}R^{4b})ₘ or C(O), and Y is O(CR^{4e}R^{4f})ₙ or NR⁵(CR^{4c}R^{4d})ᵣ; preferably, X is (CR^{4a}R^{4b})ₘ and Y is O(CR^{4e}R^{4f})ₙ, or X is (CR^{4a}R^{4b})ₘ and Y is NR⁵(CR^{4c}R^{4d})ᵣ, or X is C(O) and Y is NR⁵(CR^{4c}R^{4d})ᵣ; wherein R^{4a}, R^{4b}, R^{4c}, R^{4d}, R^{4e}, R^{4f}, R⁵, m, n, and r are as defined in general formula (IM); more preferably, X-Y is selected from the group consisting of CHzO, CH₂OCH₂, CH₂CH₂O, C(O)NH, CH₂NH, and CH₂NCH₃.

In some embodiments of the present disclosure, the compound of general formula (IM) or general formula (I) or the pharmaceutically acceptable salt thereof is provided, wherein R^{4c} and R^{4d} are identical or different and are each independently selected from the group consisting of hydrogen atom, halogen, C₁₋₆ alkyl, and C₁₋₆ haloalkyl; preferably, R^{4c} and R^{4d} are both hydrogen atoms.

In some embodiments of the present disclosure, the compound of general formula (IM) or general formula (I) or the pharmaceutically acceptable salt thereof is provided, wherein R^{4e} and R^{4f} are identical or different and are each independently selected from the group consisting of hydrogen atom, halogen, C₁₋₆ alkyl, and C₁₋₆ haloalkyl; preferably, R^{4e} and R^{4f} are both hydrogen atoms.

In some embodiments of the present disclosure, the compound of general formula (IM) or the pharmaceutically acceptable salt thereof is provided, wherein R⁰ is selected from the group consisting of hydrogen atom, halogen, C₁₋₆ alkyl, and C₁₋₆ haloalkyl; preferably, R⁰ is a hydrogen atom or halogen; more preferably, R⁰ is a hydrogen atom or F; most preferably, R⁰ is a hydrogen atom.

In some embodiments of the present disclosure, the compound of general formula (IM) or general formula (I) or the pharmaceutically acceptable salt thereof is a compound of general formula (II-1) or a pharmaceutically acceptable salt thereof: 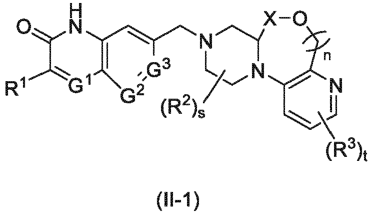 wherein:
X is (CR^{4a}R^{4b})ₘ;
G¹ to G³, R¹ to R³, R^{4a}, R^{4b}, s, t, m, and n are as defined in general formula (IM).

In some embodiments of the present disclosure, the compound of general formula (IM) or general formula (I) or the pharmaceutically acceptable salt thereof is a compound of general formula (II-2) or a pharmaceutically acceptable salt thereof: 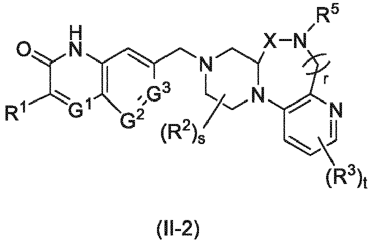 wherein:
X is (CR^{4a}R^{4b})ₘ or C(O);
G¹ to G³, R¹ to R³, R^{4a}, R^{4b}, R⁵, s, t, m, and r are as defined in general formula (IM).

In some embodiments of the present disclosure, the compound of general formula (IM), general formula (I), general formula (II-1), or general formula (II-2) or the pharmaceutically acceptable salt thereof is provided, wherein each R² is identical or different and is independently selected from the group consisting of halogen, C₁₋₆ alkyl, C₁₋₆ alkoxy, C₁₋₆ haloalkyl, and C₁₋₆ haloalkoxy.

In some embodiments of the present disclosure, the compound of general formula (IM), general formula (I), general formula (II-1), or general formula (II-2) or the pharmaceutically acceptable salt thereof is provided, wherein s is 0.

In some embodiments of the present disclosure, the compound of general formula (IM), general formula (I), general formula (II-1), or general formula (II-2) or the pharmaceutically acceptable salt thereof is provided, wherein G¹ is CH, G² is a nitrogen atom, and G³ is CR⁶; or G¹ and G² are both CH, and G³ is a nitrogen atom; or G¹ is a nitrogen atom, G² is CH, and G³ is CR⁶; preferably, G¹ is CH, G² is a nitrogen atom, and G³ is CR⁶; wherein R⁶ is as defined in general formula (IM).

In some embodiments of the present disclosure, the compound of general formula (IM), general formula (I), general formula (II-1), or general formula (II-2) or the pharmaceutically acceptable salt thereof is provided, wherein G¹ is CH, G² is a nitrogen atom, and G³ is CR⁶; or G¹ and G² are both CH, and G³ is a nitrogen atom; or G¹ is a nitrogen atom, G² is CH, and G³ is CR⁶; wherein R⁶ is as defined in general formula (IM); preferably, G¹ is CH, G² is a nitrogen atom, and G³ is CR⁶; or G¹ is a nitrogen atom, G² is CH, and G³ is CR⁶; wherein R⁶ is as defined in general formula (IM).

In some embodiments of the present disclosure, the compound of general formula (IM), general formula (I), general formula (II-1), or general formula (II-2) or the pharmaceutically acceptable salt thereof is provided, wherein t is 1 or 2; preferably, t is 1.

In some embodiments of the present disclosure, the compound of general formula (IM), general formula (I), general formula (II-1), or general formula (II-2) or the pharmaceutically acceptable salt thereof is provided, wherein R³ is selected from the group consisting of halogen, C₁₋₆ alkyl, C₁₋₆ alkoxy, C₁₋₆ haloalkyl, C₁₋₆ haloalkoxy, C₁₋₆ hydroxyalkyl, cyano, -NR^{7a}R^{7b}, hydroxy, -C(O)R⁸, -C(O)OR⁸, and -C(O)NR^{7a}R^{7b}; preferably, R³ is -C(O)NR^{7a}R^{7b}; wherein R^{7a}, R^{7b}, and R⁸ are as defined in general formula (IM); more preferably, R³ is -C(O)NHCH₃.

In some embodiments of the present disclosure, the compound of general formula (IM), general formula (I), general formula (II-1), or general formula (II-2) or the pharmaceutically acceptable salt thereof is provided, wherein m is 1 or 2.

In some embodiments of the present disclosure, the compound of general formula (IM), general formula (I), general formula (II-1), or general formula (II-2) or the pharmaceutically acceptable salt thereof is provided, wherein R^{4a} and R^{4b} are identical or different and are each independently selected from the group consisting of hydrogen atom, halogen, C₁₋₆ alkyl, and C₁₋₆ haloalkyl; preferably, R^{4a} and R^{4b} are both hydrogen atoms.

In some embodiments of the present disclosure, the compound of general formula (IM), general formula (I), or general formula (II-2) or the pharmaceutically acceptable salt thereof is provided, wherein r is 0 or 1; preferably, r is 0.

In some embodiments of the present disclosure, the compound of general formula (IM), general formula (I), or general formula (II-1) or the pharmaceutically acceptable salt thereof is a compound of general formula (III-1) or a pharmaceutically acceptable salt thereof: 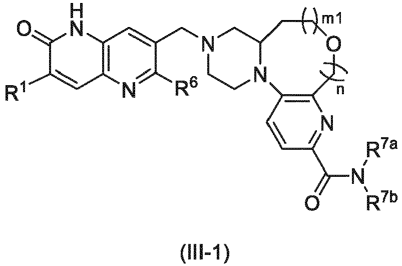 wherein:
m1 is 0 or 1;
R1, R⁶, R^{7a}, R^{7b}, and n are as defined in general formula (IM).

In some embodiments of the present disclosure, the compound of general formula (IM), general formula (I), general formula (II-1), or general formula (III-1) or the pharmaceutically acceptable salt thereof is a compound of general formula (III-1-A) or a pharmaceutically acceptable salt thereof: 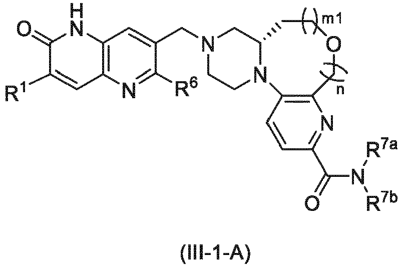 wherein:
m1 is 0 or 1;
R¹, R⁶, R^{7a}, R^{7b}, and n are as defined in general formula (IM).

In some embodiments of the present disclosure, the compound of general formula (IM), general formula (I), or general formula (II-2) or the pharmaceutically acceptable salt thereof is a compound of general formula (III-2) or a pharmaceutically acceptable salt thereof: 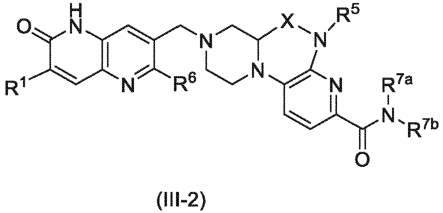 wherein:
X is CH₂ or C(O);
R¹, R⁵, R⁶, R^{7a}, and R^{7b} are as defined in general formula (IM).

In some embodiments of the present disclosure, the compound of general formula (IM), general formula (I), general formula (II-1), general formula (II-2), general formula (III-1), general formula (III-1-A), or general formula (III-2) or the pharmaceutically acceptable salt thereof is provided, wherein R¹ is selected from the group consisting of hydrogen atom, halogen, C₁₋₆ alkyl, C₁₋₆ alkoxy, C₁₋₆ haloalkyl, C₁₋₆ haloalkoxy, and C₁₋₆ hydroxyalkyl; preferably, R¹ is C₁₋₆ alkyl; more preferably, R¹ is ethyl.

In some embodiments of the present disclosure, the compound of general formula (IM), general formula (I), general formula (II-1), general formula (II-2), general formula (III-1), general formula (III-1-A), or general formula (III-2) or the pharmaceutically acceptable salt thereof is provided, wherein R⁶ is selected from the group consisting of hydrogen atom, halogen, C₁₋₆ alkyl, C₁₋₆ alkoxy, C₁₋₆ haloalkyl, C₁₋₆ haloalkoxy, and C₁₋₆ hydroxyalkyl; preferably, R⁶ is a hydrogen atom.

In some embodiments of the present disclosure, the compound of general formula (IM), general formula (I), general formula (II-1), general formula (II-2), general formula (III-1), general formula (III-1-A), or general formula (III-2) or the pharmaceutically acceptable salt thereof is provided, wherein R^{7a} and R^{7b} are identical or different and are each independently selected from the group consisting of hydrogen atom, C₁₋₆ alkyl, C₁₋₆ hydroxyalkyl, 3- to 8-membered cycloalkyl, and 3- to 8-membered heterocyclyl; preferably, R^{7a} and R^{7b} are identical or different and are each independently a hydrogen atom or C₁₋₆ alkyl; more preferably, R^{7a} is a hydrogen atom, and R^{7b} is methyl.

In some embodiments of the present disclosure, the compound of general formula (IM), general formula (I), general formula (II-1), general formula (III-1), or general formula (III-1-A) or the pharmaceutically acceptable salt thereof is provided, wherein n is 0 or 1. In some embodiments of the present disclosure, the compound of general formula (IM), general formula (I), general formula (II-2), or general formula (III-2) or the pharmaceutically acceptable salt thereof is provided, wherein R⁵ is a hydrogen atom or C₁₋₆ alkyl; preferably, R⁵ is a hydrogen atom or methyl.

In some embodiments of the present disclosure, the compound of general formula (IM) or the pharmaceutically acceptable salt thereof is provided, wherein X is (CR^{4a}R^{4b})ₘ or C(O), and Y is O(CR^{4e}R^{4f})ₙ or NR⁵(CR^{4c}R^{4d})ᵣ; G¹ is CH, G² is a nitrogen atom, and G³ is CR⁶; or G¹ is a nitrogen atom, G² is CH, and G³ is CR⁶; R⁰ is a hydrogen atom or halogen; R¹ is C₁₋₆ alkyl; R³ is -C(O)NR^{7a}R^{7b}; R^{4a}, R^{4b}, R^{4c}, R^{4d}, R^{4e}, and R^{4f} are all hydrogen atoms; R⁵ is a hydrogen atom or C₁₋₆ alkyl; R⁶ is a hydrogen atom; R^{7a} and R^{7b} are identical or different and are each independently a hydrogen atom or C₁₋₆ alkyl; m is 1 or 2; n is 0 or 1; r is 0 or 1; s is 0; t is 1.

In some embodiments of the present disclosure, the compound of general formula (I) or the pharmaceutically acceptable salt thereof is provided, wherein X is (CR^{4a}R^{4b})ₘ or C(O), and Y is O(CR^{4e}R^{4f})ₙ or NR⁵(CR^{4c}R^{4d})ᵣ; G¹ is CH, G² is a nitrogen atom, and G³ is CR⁶; or G¹ and G² are CH, and G³ is a nitrogen atom; or G¹ is a nitrogen atom, G² is CH, and G³ is CR⁶; R¹ is selected from the group consisting of hydrogen atom, halogen, C₁₋₆ alkyl, C₁₋₆ alkoxy, C₁₋₆ haloalkyl, C₁₋₆ haloalkoxy, and C₁₋₆ hydroxyalkyl; R³ is -C(O)NR^{7a}R^{7b}; R^{4a}, R^{4b}, R^{4c}, R^{4d}, R^{4e}, and R^{4f} are all hydrogen atoms; R⁵ is a hydrogen atom or C₁₋₆ alkyl; R⁶ is selected from the group consisting of hydrogen atom, halogen, C₁₋₆ alkyl, C₁₋₆ alkoxy, C₁₋₆ haloalkyl, C₁₋₆ haloalkoxy, and C₁₋₆ hydroxyalkyl; R^{7a} and R^{7b} are identical or different and are each independently selected from the group consisting of hydrogen atom, C₁₋₆ alkyl, C₁₋₆ hydroxyalkyl, 3- to 8-membered cycloalkyl, and 3- to 8-membered heterocyclyl; m is 1 or 2; n is 0 or 1; r is 0 or 1; s is 0; t is 1 or 2.

In some embodiments of the present disclosure, the compound of general formula (II-1) or the pharmaceutically acceptable salt thereof is provided, wherein X is (CR^{4a}R^{4b})ₘ; G¹ is CH, G² is a nitrogen atom, and G³ is CR⁶; R¹ is C₁₋₆ alkyl; R³ is -C(O)NR^{7a}R^{7b}; R^{4a} and R^{4b} are both hydrogen atoms; R⁶ is a hydrogen atom; R^{7a} and R^{7b} are identical or different and are each independently selected from the group consisting of hydrogen atom, C₁₋₆ alkyl, C₁₋₆ hydroxyalkyl, 3- to 8-membered cycloalkyl, and 3- to 8-membered heterocyclyl; s is 0; t is 1; m is 1 or 2; n is 0 or 1.

In some embodiments of the present disclosure, the compound of general formula (II-2) or the pharmaceutically acceptable salt thereof is provided, wherein X is (CR^{4a}R^{4b})ₘ or C(O); G¹ is CH, G² is a nitrogen atom, and G³ is CR⁶; R¹ is C₁₋₆ alkyl; R³ is -C(O)NR^{7a}R^{7b}; R^{4a} and R^{4b} are both hydrogen atoms; R⁵ is a hydrogen atom or C₁₋₆ alkyl; R⁶ is a hydrogen atom; R^{7a} and R^{7b} are identical or different and are each independently selected from the group consisting of hydrogen atom, C₁₋₆ alkyl, C₁₋₆ hydroxyalkyl, 3- to 8-membered cycloalkyl, and 3- to 8-membered heterocyclyl; s is 0; t is 1; m is 1 or 2; r is 0.

In some embodiments of the present disclosure, the compound of general formula (III-1) or general formula (III-1-A) or the pharmaceutically acceptable salt thereof is provided, wherein R¹ is C₁₋₆ alkyl; R⁶ is a hydrogen atom; R^{7a} and R^{7b} are identical or different and are each independently a hydrogen atom or C₁₋₆ alkyl; m1 is 0 or 1; n is 0 or 1.

In some embodiments of the present disclosure, the compound of general formula (III-2) or the pharmaceutically acceptable salt thereof is provided, wherein X is CH₂ or C(O); R¹ is C₁₋₆ alkyl; R⁵ is a hydrogen atom or C₁₋₆ alkyl; R⁶ is a hydrogen atom; R^{7a} and R^{7b} are identical or different and are each independently a hydrogen atom or C₁₋₆ alkyl.

**Table A. Typical compounds of the present disclosure include, but are not limited to:**

| Example No. | Compound structure | Name |
|---|---|---|
| 1 | 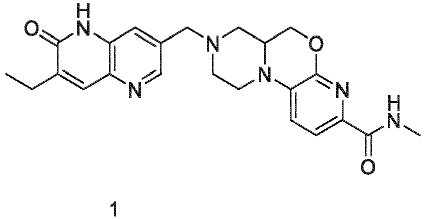 | (±)-3-((7-Ethyl-6-oxo-5,6-d ihydro-1,5-naphthyridin-3-y l)methyl)-*N-*methyl-1,2,3,4, 4*a*,5-hexahydropyrazino[1, 2-*d*]pyrido[2,3-*b*][1,4]oxazi ne-8-carboxamide **1** |
| | 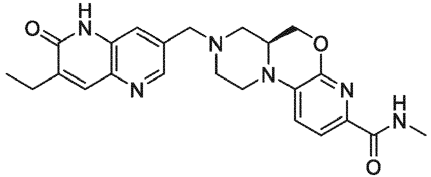 | (*S*)-3-((7-Ethyl-6-oxo-5,6-di hydro-1,5-naphthyridin-3-yl )methyl)-*N*-methyl-1,2,3,4, 4*a*,5-hexahydropyrazino[1, 2-*d*]pyrido[2,3-*b*][1,4]oxazi ne-8-carboxamide |
| 2 | 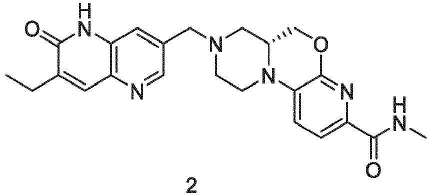 | (*R*)-3-((7-Ethyl-6-oxo-5,6-d ihydro-1,5-naphthyridin-3-y l)methyl)-*N*-methyl-1,2,3,4, 4*a*,5-hexahydropyrazino[1, 2-*d*]pyrido[2,3-*b*][1,4]oxazi ne-8-carboxamide **2** |
| 3 | 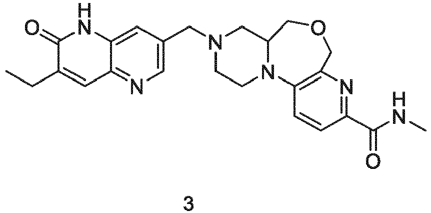 | (±)-3-((7-Ethyl-6-oxo-5,6-d ihydro-1,5-naphthyridin-3-y l)methyl)-*N-*methyl-1,2,3,4, 4*a*,5-hexahydro-7*H*-pyrazin o[2,1-*c*]pyrido[3,2-*e*][1,4]o xazepine-9-carboxamide **3** |
| | 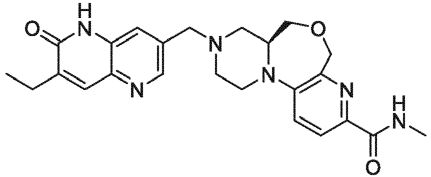 | (*S*)-3-((7-Ethyl-6-oxo-5,6-di hydro-1,5-naphthyridin-3-yl )methyl)-*N*-methyl-1,2,3,4, 4*a*,5-hexahydro-7*H*-pyrazin o[2,1-*c*]pyrido[3,2-*e*][1,4]o xazepine-9-carboxamide |
| 4 | 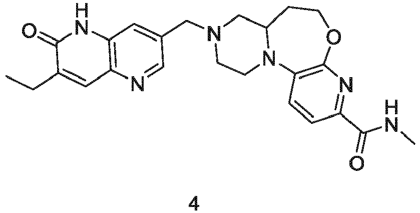 | (±)-3-((7-Ethyl-6-oxo-5,6-d ihydro-1,5-naphthyridin-3-y l)methyl)-*N*-methyl-2,3,4,4 *a*,5,6-he*x*ahydro-1*H*-pyrazi no[1,2-*d*]pyrido[2,3-*b*][1,4] oxazepine-9-carboxamide **4** |
| | 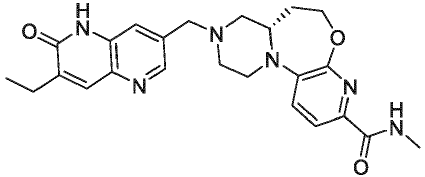 | (*S*)-3-((7-Ethyl-6-oxo-5,6-di hydro-1,5-naphthyridin-3-yl )methyl)-*N*-methyl-2,3,4,4*a* ,5,6-hexahydro-1*H*-pyrazin o[1,2-*d*]pyrido[2,3-*b*][1,4]o xazepine-9-carboxamide |
| | 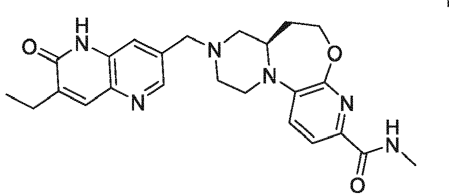 | (*R*)-3-((7-Ethyl-6-oxo-5,6-d ihydro-1,5-naphthyridin-3-y l)methyl)-*N*-methyl-2,3,4,4 *a*,5,6-hexahydro-1*H*-pyrazi no[1,2-*d*]pyrido[2,3-*b*][1,4] oxazepine-9-carboxamide |
| 5 | 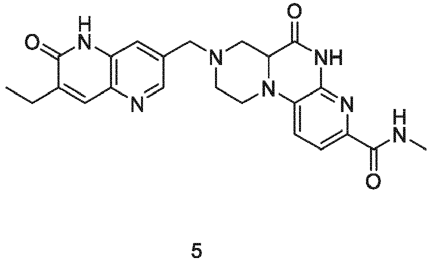 | (±)-3-((7-Ethyl-6-oxo-5,6-d ihydro-1,5-naphthyridin-3-y l)methyl)-*N-*methyl-5-oxo-2 ,3,4,4*a*,5,-hexahydro-1*H*-p yrazino[1,2-*a*]pyrido[2,3-*e*] pyrazine-8-carboxamide **5** |
| | 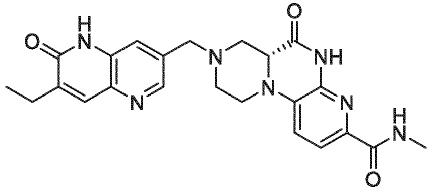 | (*R*)-3-((7-Ethyl-6-oxo-5,6-d ihydro-1,5-naphthyridin-3-y l)methyl)-*N-*methyl-5-oxo-2 ,3,4,4*a*,5,6-hexahydro-1*H*-p yrazino[1,2-*a*]pyrido[2,3-*e*] pyrazine-8-carboxamide |
| | 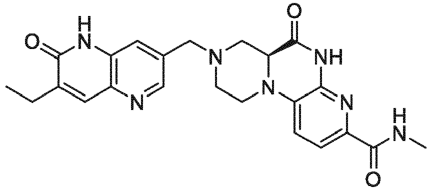 | (*S*)-3-((7-Ethyl-6-oxo-5,6-di hydro-1,5-naphthyridin-3-yl )methyl)-*N-*methyl-5-oxo-2, 3,4,4*a*,5,6-hexahydro-1*H*-p yrazino[1,2-*a*]pyrido[2,3-*e*] pyrazine-8-carboxamide |
| 6 | 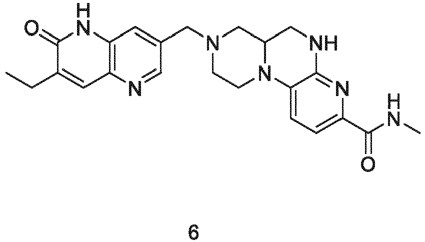 | (±)-3-((7-Ethyl-6-oxo-5,6-d ihydro-1,5-naphthyridin-3-y l)methyl)-*N-*methyl-2,3,4,4 *a*,5,6-hexahydro-1*H*-pyrazi no[1,2-*a*]pyrido[2,3-*e*]pyraz ine-8-carboxamide **6** |
| | 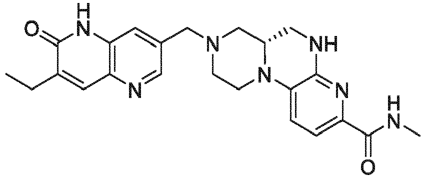 | (*S*)-3-((7-Ethyl-6-oxo-5,6-di hydro-1,5-naphthyridin-3-yl )methyl)-*N*-methyl-2,3,4,4*a* ,5,6-hexahydro-1*H*-pyrazin of 1,2-*a*]pyrido[2,3-*e*]pyrazi ne-8-carboxamide |
| | 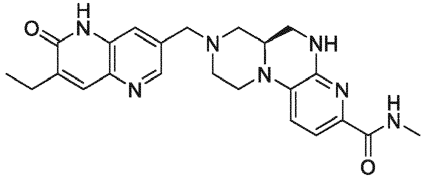 | (*R*)-3-((7-Ethyl-6-oxo-5,6-d ihydro-1,5-naphthyridin-3-y l)methyl)-*N*methyl-2,3,4,4 *a*,5,6-hexahydro-1*H-*pyrazi no[1,2-*a*]pyrido[2,3-*e*]pyraz ine-8-carboxamide |
| 7 | 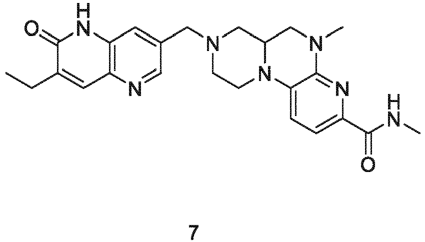 | (±)-3-((7-Ethyl-6-oxo-5,6-d ihydro-1,5-naphthyridin-3-y l)methyl)-*N*,6-dimethyl-2,3, 4,4*a*,5,6-hexahydro-1*H*-pyr azino[1,2-*a*]pyrido[2,3-*e*]py razine-8-carboxamide 7 |
| | 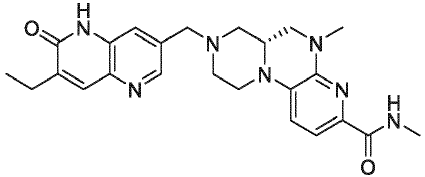 | (*R*)-3-((7-Ethyl-6-oxo-5,6-d ihydro-1,5-naphthyridin-3-y l)methyl)-*N*,6-dimethyl-2,3, 4,4*a*,5,6-hexahydro-1*H*-pyr azino[1,2-*a*]pyrido[2,3-*e*]py razine-8-carboxamide |
| | 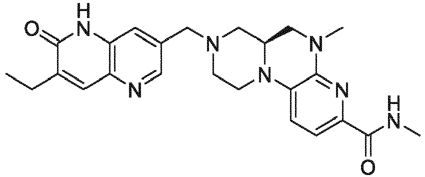 | (*S*)-3-((7-Ethyl-6-oxo-5,6-di hydro-1,5-naphthyridin-3-yl )methyl)-*N*,6-dimethyl-2,3, 4,4*a*,5,6-hexahydro-1*H*-pyr azino[1,2-*a*]pyrido[2,3-*e*]py razine-8-carboxamide |
| 8 | 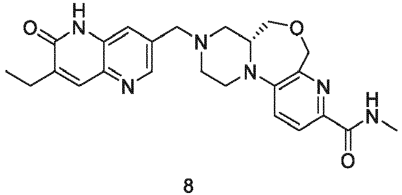 | (*R*)-3-((7-Ethyl-6-oxo-5,6-d ihydro-1,5-naphthyridin-3-y l)methyl)-*N*-methyl-1,2,3,4, 4*a*,5-hexahydro-7*H*-pyrazin o[2,1-*c*]pyrido[3,2-*e*][1,4]o xazepine-9-carboxamide **8** |
| 9 | 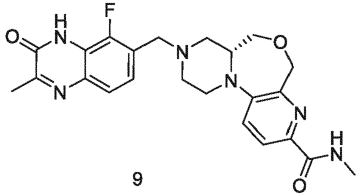 | (*R*)-3-((5-Fluoro-2-methyl-3-oxo-3,4-dihydroquinoxali n-6-yl)methyl)-*N*-methyl-1, 2,3,4,4*a*,5-hexahydro-7*H*-p yrazino[2,1-*c*]pyrido[3,2-*e*][ 1,4]oxazepine-9-carboxami de **9** |
| | 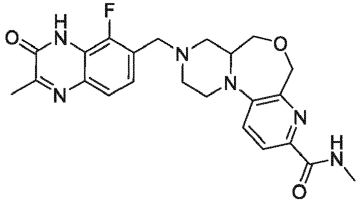 | (±)-3-((5-Fluoro-2-methyl-3 -oxo-3,4-dihydroquinoxalin -6-yl)methyl)-*N*-methyl-1,2, 3,4,4*a*,5-hexahydro-7*H*-pyr azino[2,1-*c*]pyrido[3,2-*e*][1, 4]oxazepine-9-carboxamide |
| | 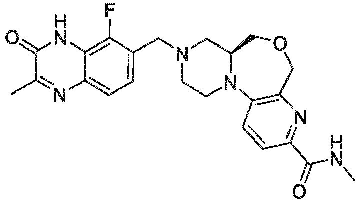 | (*S*)-3-((5 -Fluoro-2-methyl-3 -oxo-3,4-dihydroquinoxalin -6-yl)methyl)-*A*-methyl-1,2, 3,4,4*a*,5-hexahydro-7*H*-pyr azino[2,1-*c*]pyrido[3,2-*e*][1, 4]oxazepine-9-carboxamide |
| 10 | 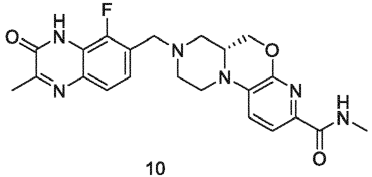 | (*R*)-3-((5-Fluoro-2-methyl-3-oxo-3,4-dihydroquinoxali n-6-yl)methyl)-*N-*methyl-1, 2,3,4,4*a*,5-hexahydropyrazi no[1,2-*d*]pyrido[2,3-*b*][1,4] oxazine-8-carboxamide **10** |
| | 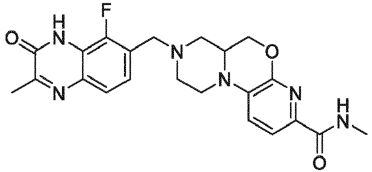 | (±)-3-((5-Fluoro-2-methyl-3 -oxo-3,4-dihydroquinoxalin -6-yl)methyl)-*N*-methyl-1,2, 3,4,4*a*,5-hexahydropyrazino [1,2-*d*]pyrido[2,3-*b*][1,4]ox azine-8-carboxamide |
| | 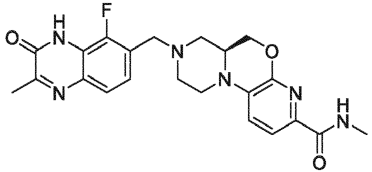 | (*S*)-3-((5-Fluoro-2-methyl-3 -oxo-3,4-dihydroquinoxalin -6-yl)methyl)-*N*-methyl-1,2, 3,4,4*a*,5-hexahydropyrazino [1,2-*d*]pyrido[2,3-*b*][1,4]ox azine-8-carboxamide |

Another aspect of the present disclosure relates to a compound of general formula (IMa) or a salt thereof: 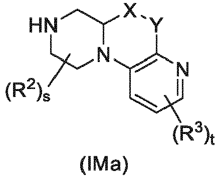 wherein:
t is 1, 2, or 3;
X, Y, R², R³, and s are as defined in the compound of general formula (IM).

Another aspect of the present disclosure relates to a compound of general formula (II-1a) or a salt thereof: 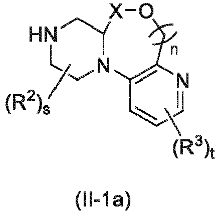 wherein:
X, R², R³, s, t, and n are as defined in the compound of general formula (II-1).

Another aspect of the present disclosure relates to a compound of general formula (II-2a) or a salt thereof: 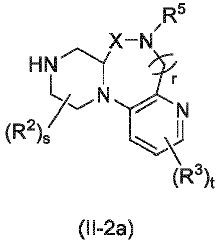 wherein:
X, R², R³, R⁵, s, t, and r are as defined in the compounds of general formula (II-2).

Another aspect of the present disclosure relates to a compound of general formula (III-1a) or a salt thereof: 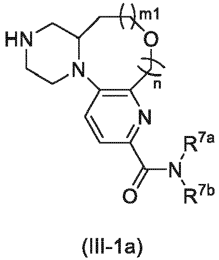 wherein:
m1 is 0 or 1;
R^{7a}, R^{7b}, and n are as defined in the compound of general formula (III-1).

Another aspect of the present disclosure relates to a compound of general formula (III-1-Aa) or a salt thereof: 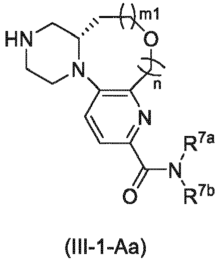 wherein:
m1 is 0 or 1;
R^{7a}, R^{7b}, and n are as defined in the compound of general formula (III-1-A).

Another aspect of the present disclosure relates to a compound of general formula (III-2a) or a salt thereof: 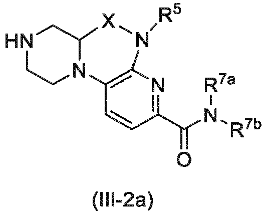 wherein:
X, R⁵, R^{7a}, and R^{7b} are as defined in general formula (III-2).

**Table B. Typical intermediate compounds of the present disclosure include, but are not limited to:**

| Compound No. | Compound structure | Name |
|---|---|---|
| 1h | 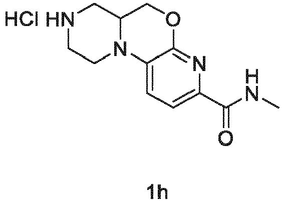 | (±)-*N*-Methyl-1,2,3,4,4*a*,5-hexahydro pyrazino[1,2-*d*]pyrido[2,3-*b*][1,4]oxaz ine-8-carboxamide hydrochloride **1h** |
| 2f | 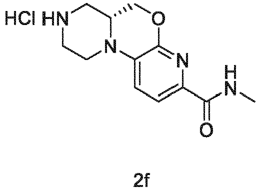 | (*R*)-*N-*Methyl-1,2,3,4,4*a*,5-hexahydro pyrazino[1,2-*d*]pyrido[2,3-*b*][1,4]oxaz ine-8-carboxamide hydrochloride **2f** |
| | 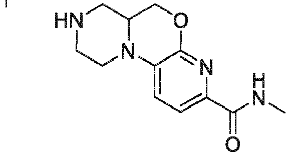 | (±)-*N*-Methyl-1,2,3,4,4*a*,5-hexahydro pyrazino[1,2-*d*]pyrido[2,3-*b*][1,4]oxaz ine-8-carboxamide |
| | 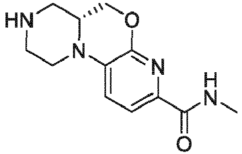 | (*R*)-*N-*Methyl-1,2,3,4,4*a*,5-hexahydro pyrazino[1,2-*d*]pyrido[2,3-*b*][1,4]oxaz ine-8-carboxamide |
| | 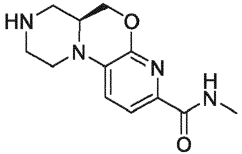 | (*S*)-*N*-Methyl-1,2,3,4,4*a*,5-hexahydro pyrazino[1,2-*d*]pyrido[2,3-*b*][1,4]oxaz ine-8-carboxamide |
| | 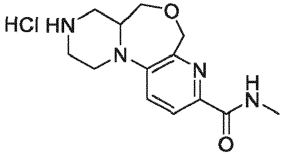 | (±)-*N*-Methyl-1,2,3,4,4*a*,5-hexahydro-7*H*-pyrazino[2,1-*c*]pyrido[3,2-*e*][1,4]o xazepine-9-carboxamide hydrochloride |
| | 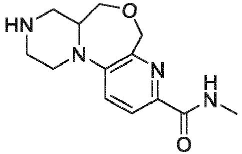 | (±)-*N*-Methyl-1,2,3,4,4*a*,5-hexahydro-7H-pyrazino[2,1-*c*]pyrido[3,2-*e*][1,4]o xazepine-9-carboxamide |
| | 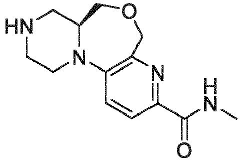 | (*S*)-*N*-Methyl-1,2,3,4,4*a*,5-hexahydro-7*H*-pyrazino[2,1-*c*]pyrido[3,2-*e*][1,4]o xazepine-9-carboxamide |
| | 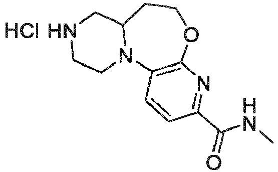 | (±)-*N*-Methyl-2,3,4,4*a*,5,6-hexahydro-1*H*-pyrazino[1,2-*d*]pyrido[2,3-*b*][1,4] oxazepine-9-carboxamide hydrochloride |
| | 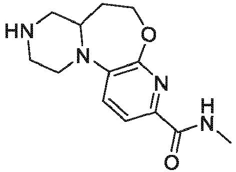 | (±)-*N*-Methyl-2,3,4,4*a*,5,6-hexahydro-1*H*-pyrazino[1,2-*d*]pyrido[2,3-*b*][1,4] oxazepine-9-carboxamide |
| | 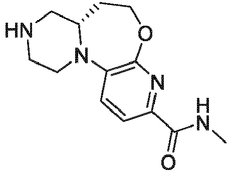 | (*S*)-*N*-Methyl-2,3,4,4*a*,5,6-hexahydro-1*H*-pyrazino[1,2-*d*]pyrido[2,3-*b*][1,4] oxazepine-9-carboxamide |
| | 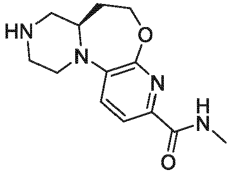 | (*R*)-*N-*Methyl-2,3,4,4*a*,5,6-hexahydro-1*H*-pyrazino[1,2-*d*]pyrido[2,3-*b*][1,4] oxazepine-9-carboxamide |
| 5e | 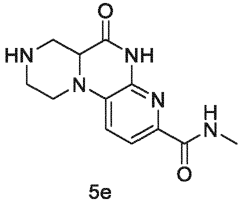 | (±)-*N*-Methyl-5-oxo-2,3,4,4*a*,5,6-hexa hydro-1*H*-pyrazino[1,2-*a*]pyrido[2,3-*e* ]pyrazine-8-carboxamide **5e** |
| | 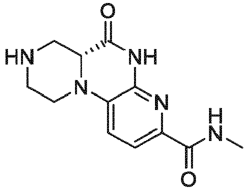 | (*R*)-*N*-Methyl-5-oxo-2,3,4,4*a*,5,6-hexa hydro-1*H*-pyrazino[1,2-*a*]pyrido[2,3-*e* ]pyrazine-8-carboxamide |
| | 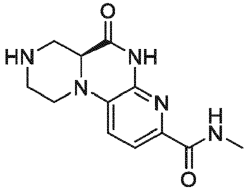 | (*S*)-*N*-Methyl-5-oxo-2,3,4,4*a*,5,6-hexa hydro-1*H*-pyrazino[1,2-*a*]pyrido[2,3-*e* ]pyrazine-8-carboxamide |
| | 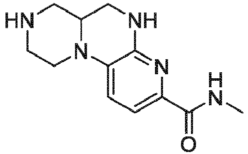 | (±)-*N*-Methyl-2,3,4,4*a*,5,6-hexahydro-1*H*-pyrazino[1,2-*a*]pyrido[2,3-*e*]pyraz ine-8-carboxamide |
| | 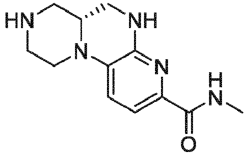 | (*R*)-*N*-Methyl-2,3,4,4*a*,5,6-hexahydro-1*H*-pyrazino[1,2-*a*]pyrido[2,3-*e*]pyraz ine-8-carboxamide |
| | 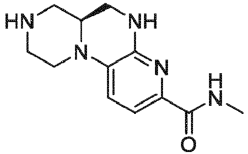 | (*S*)-*N*-Methyl-2,3,4,4*a*,5,6-hexahydro-1*H*-pyrazino[1,2-*a*]pyrido[2,3-*e*]pyraz ine-8-carboxamide |
| | 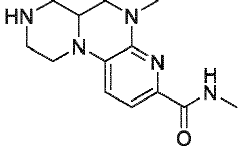 | (±)-*N*,6-Dimethyl-2,3,4,4a,5,6-hexahy dro-1*H*-pyrazino[1,2-*a*]pyrido[2,3-*e*]p yrazine-8-carboxamide |
| | 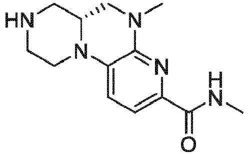 | (*R*)-A,6-Dimethyl-2,3,4,4a,5,6-hexahy dro-1*H*-pyrazino[1,2-*a*]pyrido[2,3-*e*]p yrazine-8-carboxamide |
| | 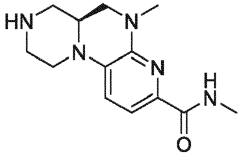 | (*S*)-*N*,6-Dimethyl-2,3,4,4a,5,6-hexahy dro-1*H*-pyrazino[1,2-*a*]pyrido[2,3-*e*]p yrazine-8-carboxamide |
| 8f | 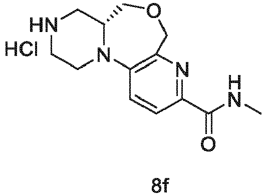 | (*R*)-*N*-Methyl-1,2,3,4,4*a*,5-hexahydro-7*H*-pyrazino[2,1-*c*]pyrido[3,2-*e*][1,4]o xazepine-9-carboxamide hydrochloride **8f** |
| | 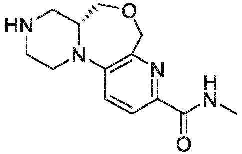 | (*R*)-*N*-Methyl-1,2,3,4,4*a*,5-hexahydro-7*H*-pyrazino[2,1-*c*]pyrido[3,2-*e*][1,4]o xazepine-9-carboxamide |

Another aspect of the present disclosure relates to a method for preparing a compound of general formula (IM) or a pharmaceutically acceptable salt thereof, which comprises: 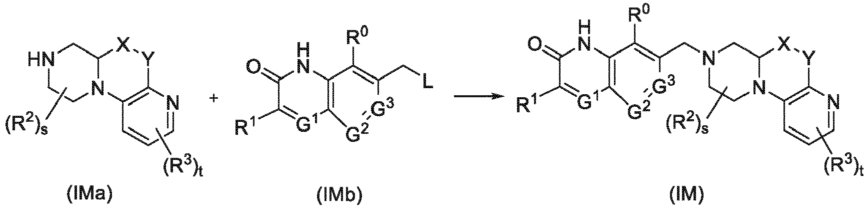
conducting a nucleophilic substitution reaction of a compound of general formula (IMa) or a salt (preferably hydrochloride) thereof with a compound of general formula (IMb) to give the compound of general formula (IM) or the pharmaceutically acceptable salt thereof;
wherein:
   L is halogen, preferably a chlorine atom;
   X, Y, G¹ to G³, R⁰ to R³, s, and t are as defined in general formula (IM).

Another aspect of the present disclosure relates to a method for preparing a compound of general formula (I) or a pharmaceutically acceptable salt thereof, which comprises: 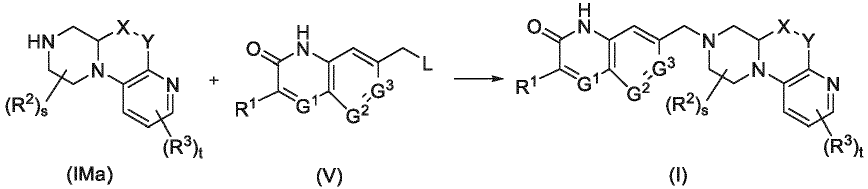
conducting a nucleophilic substitution reaction of a compound of general formula (IMa) or a salt (preferably hydrochloride) thereof with a compound of general formula (V) to give the compound of general formula (I) or the pharmaceutically acceptable salt thereof;
wherein:
   L is halogen, preferably a chlorine atom;
   X, Y, G¹ to G³, R¹ to R³, s, and t are as defined in general formula (I).

Another aspect of the present disclosure relates to a method for preparing a compound of general formula (11-1) or a pharmaceutically acceptable salt thereof, which comprises: 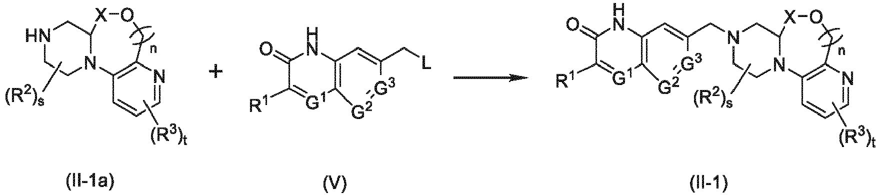
conducting a nucleophilic substitution reaction of a compound of general formula (II-1a) or a salt (preferably hydrochloride) thereof with a compound of general formula (V) to give the compound of general formula (11-1) or the pharmaceutically acceptable salt thereof;
wherein:
   L is halogen, preferably a chlorine atom;
   X, G¹ to G³, R¹ to R³, s, t, and n are as defined in general formula (II-1).

Another aspect of the present disclosure relates to a method for preparing a compound of general formula (II-2) or a pharmaceutically acceptable salt thereof, which comprises: 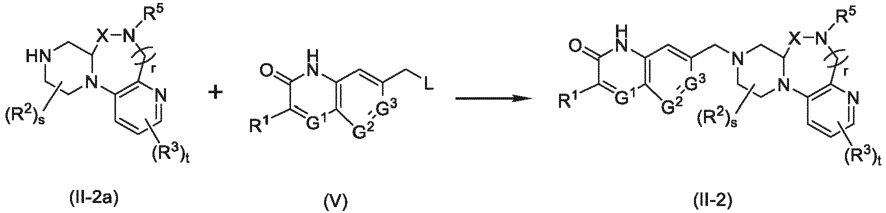
conducting a nucleophilic substitution reaction of a compound of general formula (II-2a) or a salt (preferably hydrochloride) thereof with a compound of general formula (V) to give the compound of general formula (II-2) or the pharmaceutically acceptable salt thereof;
wherein:
   L is halogen, preferably a chlorine atom;
   X, G¹ to G³, R¹ to R³, R⁵, s, t, and r are as defined in general formula (II-2).

Another aspect of the present disclosure relates to a method for preparing a compound of general formula (III-1) or a pharmaceutically acceptable salt thereof, which comprises: 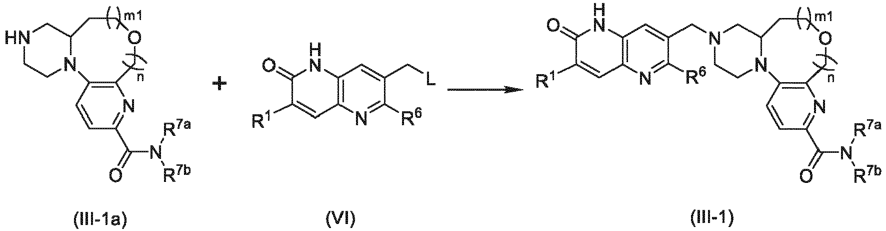
conducting a nucleophilic substitution reaction of a compound of general formula (III-1a) or a salt (preferably hydrochloride) thereof with a compound of general formula (VI) to give the compound of general formula (III-1) or the pharmaceutically acceptable salt thereof;
wherein:
   m1 is 0 or 1;
   L is halogen, preferably a chlorine atom;
   R¹, R⁶, R^{7a}, R^{7b}, and n are as defined in general formula (III-1).

Another aspect of the present disclosure relates to a method for preparing a compound of general formula (III-1-A) or a pharmaceutically acceptable salt thereof, which comprises: 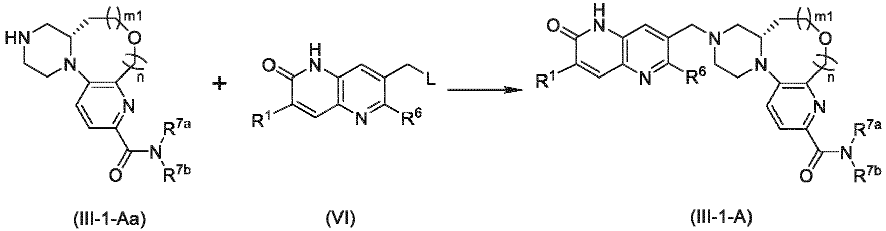
conducting a nucleophilic substitution reaction of a compound of general formula (III-1-Aa) or a salt (preferably hydrochloride) thereof with a compound of general formula (VI) to give the compound of general formula (III-1-A) or the pharmaceutically acceptable salt thereof;
wherein:
   m1 is 0 or 1;
   L is halogen, preferably a chlorine atom;
   R¹, R⁶, R^{7a}, R^{7b}, and n are as defined in general formula (III-1-A).

Another aspect of the present disclosure relates to a method for preparing a compound of general formula (III-2) or a pharmaceutically acceptable salt thereof, which comprises: 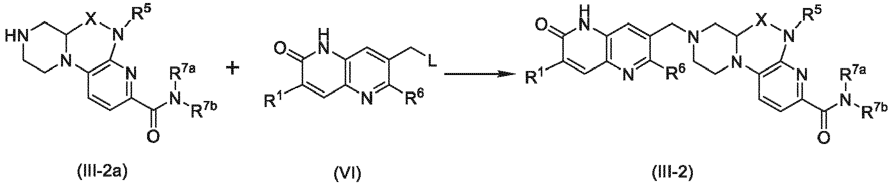
conducting a nucleophilic substitution reaction of a compound of general formula (III-2a) or a salt (preferably hydrochloride) thereof with a compound of general formula (VI) to give the compound of general formula (III-2) or the pharmaceutically acceptable salt thereof;
wherein:
   L is halogen, preferably a chlorine atom;
   X, R¹, R⁵, R⁶, R^{7a}, and R^{7b} are as defined in general formula (III-2).

Another aspect of the present disclosure relates to a pharmaceutical composition comprising the compound of general formula (IM), general formula (I), general formula (II-1), general formula (II-2), general formula (III-1), general formula (III-1-A), or general formula (III-2) of the present disclosure and a compound shown in Table A or a pharmaceutically acceptable salt thereof, and one or more pharmaceutically acceptable carriers, diluents, or excipients.

The present disclosure further relates to use of the compound of general formula (IM), general formula (I), general formula (II-1), general formula (II-2), general formula (III-1), general formula (III-1-A), or general formula (III-2) and a compound shown in Table A or a pharmaceutically acceptable salt thereof, or the pharmaceutical composition comprising same in the preparation of a PARP1 inhibitor.

The present disclosure further relates to use of the compound of general formula (IM), general formula (I), general formula (II-1), general formula (II-2), general formula (III-1), general formula (III-1-A), or general formula (III-2) and a compound shown in Table A or a pharmaceutically acceptable salt thereof, or the pharmaceutical composition comprising same in the preparation of a medicament for treating and/or preventing cancer.

The present disclosure further relates to a method for inhibiting PARP1, which comprises administering to a patient in need thereof an inhibitory effective amount of the compound of general formula (IM), general formula (I), general formula (II-1), general formula (II-2), general formula (III-1), general formula (III-1-A), or general formula (III-2) and a compound shown in Table A or a pharmaceutically acceptable salt thereof, or the pharmaceutical composition comprising same.

The present disclosure further relates to a method for treating and/or preventing cancer, which comprises administering to a patient in need thereof a therapeutically effective amount of the compound of general formula (IM), general formula (I), general formula (II-1), general formula (II-2), general formula (III-1), general formula (III-1-A), or general formula (III-2) and a compound shown in Table A or a pharmaceutically acceptable salt thereof, or the pharmaceutical composition comprising same.

The present disclosure further relates to a compound of general formula (IM), general formula (I), general formula (II-1), general formula (II-2), general formula (III-1), general formula (III-1-A), or general formula (III-2) and a compound shown in Table A or a pharmaceutically acceptable salt thereof, or a pharmaceutical composition comprising same for use as a medicament.

The present disclosure further relates to a compound of general formula (IM), general formula (I), general formula (II-1), general formula (II-2), general formula (III-1), general formula (III-1-A), or general formula (III-2) and a compound shown in Table A or a pharmaceutically acceptable salt thereof, or a pharmaceutical composition comprising same for use as a PARP1 inhibitor.

The present disclosure further relates to a compound of general formula (IM), general formula (I), general formula (II-1), general formula (II-2), general formula (III-1), general formula (III-1-A), or general formula (III-2) and a compound shown in Table A or a pharmaceutically acceptable salt thereof, or a pharmaceutical composition comprising same for use in treating and/or preventing cancer.

The cancer described in the present disclosure is selected from the group consisting of breast cancer, ovarian cancer, pancreatic cancer, prostate cancer, stomach cancer, colorectal cancer (e.g., colon cancer and rectal cancer), lung cancer, kidney cancer, liver cancer (e.g., hepatocellular cancer), cervical cancer, endometrial cancer, myeloma (e.g., multiple myeloma), leukemia (e.g., acute leukemia, chronic leukemia, myelogenous leukemia, myelofibrosis, and erythroleukemia), lymphoma (e.g., diffuse large B-cell lymphoma, Hodgkin's lymphoma, non-Hodgkin's lymphoma, lymphoid malignancies of T-cell or B-cell origin, and follicular lymphoma), acoustic neuroma, basal cell carcinoma, cholangiocarcinoma, bladder cancer, brain cancer, bronchial cancer, sarcoma (e.g., chondrosarcoma, fibrosarcoma, leiomyosarcoma, liposarcoma, lymphangiosarcoma, myxosarcoma, osteogenic sarcoma, and rhabdomyosarcoma), chordoma, choriocarcinoma, craniopharyngioma, cystadenocarcinoma, embryonal carcinoma, hemangioendothelioma, ependymoma, epithelial carcinoma, esophageal cancer (also referred to as esophagus cancer), essential thrombocytosis, Ewing sarcoma, testicular cancer, glioma, heavy chain disease, hemangioblastoma, medullary carcinoma, medulloblastoma, melanoma, meningioma, mesothelioma, neuroblastoma, NUT midline carcinoma, neuroglioma, bone cancer, nasopharyngeal cancer, oral cancer, thyroid cancer, pinealoma, polycythemia vera, retinoblastoma, sebaceous carcinoma, seminoma, skin cancer, squamous cell carcinoma, synovioma, sweat gland carcinoma, Waldenström macroglobulinemia, and Wilms tumor; preferably, the cancer is selected from the group consisting of breast cancer, ovarian cancer, pancreatic cancer, prostate cancer, stomach cancer, colorectal cancer, and lung cancer.

The active compound may be formulated into a form suitable for administration by any suitable route, and one or more pharmaceutically acceptable carriers are used to formulate the composition of the present disclosure by conventional methods. Thus, the active compound of the present disclosure may be formulated into a variety of dosage forms for oral administration, administration by injection (e.g., intravenous, intramuscular or subcutaneous), or administration by inhalation or insufflation. The compounds of the present disclosure may also be formulated into a dosage form, such as tablets, hard or soft capsules, aqueous or oily suspensions, emulsions, injections, dispersible powders or granules, suppositories, lozenges, or syrups.

As a general guide, the active compound is preferably in a form of a unit dose, or in a form of a single dose that can be self-administered by a patient. The unit dose of the compound or composition of the present disclosure may be in a tablet, capsule, cachet, vial, powder, granule, lozenge, suppository, regenerating powder, or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the composition may comprise 0.1 wt.% to 99 wt.% of the active compound.

The tablet comprises the active ingredient and a non-toxic pharmaceutically acceptable excipient that is used for mixing and is suitable for the preparation of the tablet. Such an excipient may be an inert excipient, a granulating agent, a disintegrant, a binder, and a lubricant. Such a tablet may be uncoated or may be coated by known techniques for masking the taste of the drug or delaying the disintegration and absorption of the drug in the gastrointestinal tract and thus enabling a sustained release of the drug over a longer period.

An oral formulation in a soft gelatin capsule where the active ingredient is mixed with an inert solid diluent or with a water-soluble carrier or oil vehicle may also be provided. An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more flavoring agents, and one or more sweeteners.

An oil suspension may be formulated by suspending the active ingredient in a vegetable oil, or in a mineral oil. The oil suspension may comprise a thickening agent. The sweeteners and flavoring agents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a flavoring agent, a preservative, and an antioxidant. Such a formulation may also comprise a palliative, a preservative, a colorant, and an antioxidant.

The pharmaceutical composition of the present disclosure may be in the form of a sterile injectable aqueous solution. Acceptable vehicles or solvents that can be used include water, Ringer's solution, and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent. In addition, a sterile fixed oil may be conveniently used as a solvent or a suspending medium. For this purpose, any blend fixed oil may be used. In addition, fatty acids may also be used to prepare injections. The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compound of the present disclosure can be administered in the form of dispersible powders and granules that are formulated into aqueous suspensions by adding water. Such a pharmaceutical composition can be prepared by mixing the active ingredient with a dispersant or a wetting agent, a suspending agent, or one or more preservatives. As is well known to those skilled in the art, the dose of the drug administered depends on a variety of factors, including but not limited to: the activity of the particular compound used, the age of the patient, the body weight of the patient, the health condition of the patient, the behavior of the patient, the diet of the patient, the time of administration, the route of administration, the rate of excretion, the combination of drugs, the severity of the disease, and the like. In addition, the optimal treatment regimen, such as the mode of administration, the daily dose of the compound, or the type of pharmaceutically acceptable salts, can be verified according to conventional treatment regimens.

### Description of the terms

Unless otherwise stated, the terms used in the specification and claims have the following meanings.

The term "alkyl" refers to a saturated aliphatic hydrocarbon group which is a linear or branched group containing 1 to 20 carbon atoms, preferably alkyl having 1 to 12 (e.g., 1, 2, 3, 4, 5, 6, 7, 8, 9, 10, 11, and 12) carbon atoms (i.e., C₁₋₁₂ alkyl), and more preferably alkyl having 1 to 6 carbon atoms (i.e., C₁₋₆ alkyl). Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof, and the like. Alkyl may be substituted or unsubstituted. When substituted, it may be substituted at any available connection site, and the substituent is optionally selected from the group consisting of one or more of D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkylene" refers to a saturated linear or branched aliphatic hydrocarbon group, which is a residue derived from the parent alkane by removal of two hydrogen atoms from the same carbon atom or two different carbon atoms. Alkylene is a linear or branched group containing 1 to 20 carbon atoms, preferably alkylene having 1 to 12 (e.g., 1, 2, 3, 4, 5, 6, 7, 8, 9, 10, 11, and 12) carbon atoms (i.e., C₁₋₁₂ alkylene), and more preferably alkylene having 1 to 6 carbon atoms (i.e., C₁₋₆ alkylene). Non-limiting examples of alkylene include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and the like. Alkylene may be substituted or unsubstituted. When substituted, it may be substituted at any available connection site, and the substituent is optionally selected from the group consisting of one or more of alkenyl, alkynyl, alkoxy, haloalkoxy, cycloalkyloxy, heterocyclyloxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo.

The term "alkenyl" refers to alkyl containing at least one carbon-carbon double bond in the molecule, wherein alkyl is as defined above; alkenyl preferably has 2 to 12 (e.g., 2, 3, 4, 5, 6, 7, 8, 9, 10, 11, and 12) carbon atoms (i.e., C₂₋₁₂ alkenyl), and more preferably has 2 to 6 carbon atoms (i.e., C₂₋₆ alkenyl). Non-limiting examples include: ethenyl, propenyl, isopropenyl, butenyl, and the like. Alkenyl may be substituted or unsubstituted. When it is substituted, the substituent is preferably selected from the group consisting of one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to alkyl containing at least one carbon-carbon triple bond in the molecule, wherein the alkyl is as defined above. Alkynyl preferably has 2 to 12 (e.g., 2, 3, 4, 5, 6, 7, 8, 9, 10, 11, and 12) carbon atoms (i.e., C₂₋₁₂ alkynyl), and more preferably has 2 to 6 carbon atoms (i.e., C₂₋₆ alkynyl). Non-limiting examples include: ethynyl, propynyl, butynyl, pentynyl, hexynyl, and the like. Alkynyl may be substituted or unsubstituted. When it is substituted, the substituent is preferably selected from the group consisting of one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic or polycyclic hydrocarbon substituent. The cycloalkyl ring contains 3 to 20 carbon atoms, preferably 3 to 12 (e.g., 3, 4, 5, 6, 7, 8, 9, 10, 11, and 12) carbon atoms (i.e., 3- to 12-membered cycloalkyl), preferably 3 to 8 carbon atoms (i.e., 3- to 8-membered cycloalkyl), and more preferably 3 to 6 carbon atoms (i.e., 3- to 6-membered cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "spiro cycloalkyl" refers to a 5- to 20-membered polycyclic group in which monocyclic rings share one carbon atom (referred to as the spiro atom), and it may contain one or more double bonds. The spiro cycloalkyl is preferably 6- to 14-membered, and more preferably 7- to 10-membered (e.g., 7-, 8-, 9-, or 10-membered). According to the number of the spiro atoms shared among the rings, the spiro cycloalkyl may be monospiro cycloalkyl or polyspiro cycloalkyl (e.g., bispiro cycloalkyl), preferably monospiro cycloalkyl and bispiro cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiro cycloalkyl. Non-limiting examples of spiro cycloalkyl include:

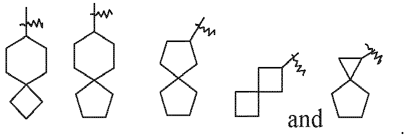

The term "fused cycloalkyl" refers to a 5- to 20-membered all-carbon polycyclic group in which each ring shares a pair of adjacent carbon atoms with the other rings in the system, wherein one or more of the rings may contain one or more double bonds. The fused cycloalkyl is preferably 6- to 14-membered, and more preferably 7- to 10-membered (e.g., 7-, 8-, 9-, or 10-membered). According to the number of constituent rings, fused cycloalkyl may be bicyclic or polycyclic (e.g., tricyclic or tetracyclic) fused cycloalkyl, preferably bicyclic or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused cycloalkyl. Non-limiting examples of fused cycloalkyl include:

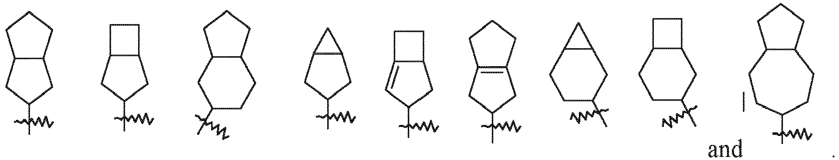

The term "bridged cycloalkyl" refers to a 5- to 20-membered all-carbon polycyclic group in which any two rings share two carbon atoms that are not directly connected, and it may contain one or more double bonds. The bridged cycloalkyl is preferably 6- to 14-membered, and more preferably 7- to 10-membered (e.g., 7-, 8-, 9-, or 10-membered). According to the number of the formed rings, the bridged cycloalkyl may be bicyclic or polycyclic (e.g., tricyclic or tetracyclic) bridged cycloalkyl, preferably bicyclic, tricyclic, or tetracyclic bridged cycloalkyl, and more preferably bicyclic or tricyclic bridged cycloalkyl. Non-limiting examples of bridged cycloalkyl include:

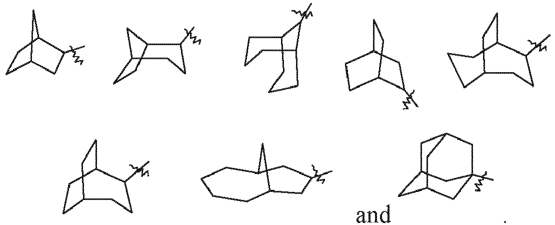

The cycloalkyl ring includes those in which the cycloalkyl described above (including monocyclic, spiro, fused, and bridged ones) is fused to an aryl, heteroaryl, or heterocycloalkyl ring, wherein the ring connected to the parent structure is cycloalkyl. Non-limiting examples include 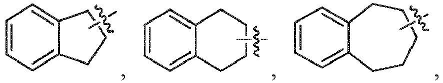 and the like, and it is preferably 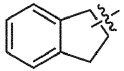 or

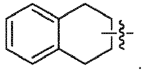

Cycloalkyl may be substituted or unsubstituted. When substituted, it may be substituted at any available connection site, and the substituent is preferably one or more substituents independently and optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples of alkoxy include: methoxy, ethoxy, propoxy, and butoxy. Alkoxy may be optionally substituted or unsubstituted. When it is substituted, the substituent is preferably selected from the group consisting of D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heterocyclyl" refers to a saturated or partially unsaturated monocyclic or polycyclic substituent containing 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl), wherein one or more ring atoms are heteroatoms selected from the group consisting of nitrogen, oxygen, and sulfur, and the sulfur may optionally be substituted with oxo (i.e., to form sulfoxide or sulfone), but does not include a cyclic portion of -O-O-, -O-S-, or -S-S-, the remaining ring atoms being carbon. Preferably, the heterocyclyl contains 3 to 12 (e.g., 3, 4, 5, 6, 7, 8, 9, 10, 11, and 12) ring atoms (i.e., 3- to 12-membered heterocyclyl), of which 1 to 4 (e.g., 1, 2, 3, and 4) are heteroatoms; more preferably, the heterocyclyl contains 3 to 8 (e.g., 3, 4, 5, 6, 7, and 8) ring atoms (i.e., 3- to 8-membered heterocyclyl), of which 1 to 3 (e.g., 1, 2, and 3) are heteroatoms; more preferably, the heterocyclyl contains 3 to 6 ring atoms, of which 1 to 3 are heteroatoms; most preferably, the heterocyclyl contains 5 or 6 ring atoms, of which 1 to 3 are heteroatoms. Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiro heterocyclyl, fused heterocyclyl, and bridged heterocyclyl.

The term "spiro heterocyclyl" refers to a 5- to 20-membered polycyclic heterocyclyl group in which monocyclic rings share one atom (referred to as the spiro atom), wherein one or more ring atoms are heteroatoms selected from the group consisting of nitrogen, oxygen, and sulfur, and the sulfur may optionally be substituted with oxo (i.e., to form sulfoxide or sulfone), the remaining ring atoms being carbon. It may contain one or more double bonds. The spiro heterocyclyl is preferably 6- to 14-membered (e.g., 6-, 7-, 8-, 9-, 10-, 11-, 12-, 13-, and 14-membered) (i.e., 6- to 14-membered spiro heterocyclyl), and more preferably 7- to 10-membered (e.g., 7-, 8-, 9-, or 10-membered) (i.e., 7- to 10-membered spiro heterocyclyl). According to the number of spiro atoms shared among the rings, the spiro heterocyclyl may be monospiro heterocyclyl or polyspiro heterocyclyl (e.g., bispiro heterocyclyl), preferably monospiro heterocyclyl and bispiro heterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiro heterocyclyl. Non-limiting examples of spiro heterocyclyl include:

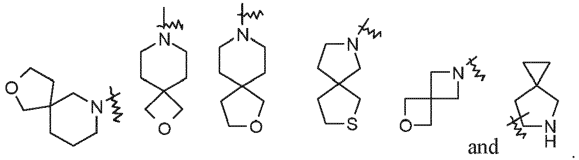

The term "fused heterocyclyl" refers to a 5- to 20-membered polycyclic heterocyclyl group in which each ring shares a pair of adjacent atoms with the other rings in the system, wherein one or more rings may contain one or more double bonds, wherein one or more ring atoms are heteroatoms selected from the group consisting of nitrogen, oxygen, and sulfur, and the sulfur may optionally be substituted with oxo (i.e., to form sulfoxide or sulfone), the remaining ring atoms being carbon. The fused heterocyclyl is preferably 6- to 14-membered (e.g., 6-, 7-, 8-, 9-, 10-, 11-, 12-, 13-, and 14-membered) (i.e., 6- to 14-membered fused heterocyclyl), and more preferably 7- to 10-membered (e.g., 7-, 8-, 9-, or 10-membered) (i.e., 7- to 10-membered fused heterocyclyl). According to the number of the formed rings, the fused heterocyclyl may be bicyclic or polycyclic (e.g., tricyclic or tetracyclic) fused heterocyclyl, preferably bicyclic or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl. Non-limiting examples of fused heterocyclyl include: 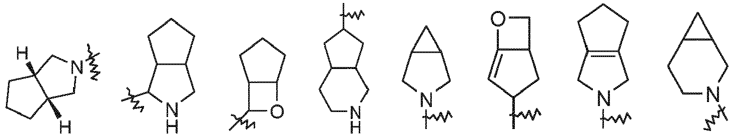 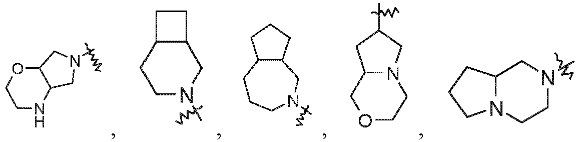 and

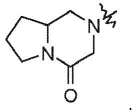

The term "bridged heterocyclyl" refers to a 5- to 14-membered polycyclic heterocyclyl group in which any two rings share two atoms that are not directly connected, and it may contain one or more double bonds, wherein one or more ring atoms are heteroatoms selected from the group consisting of nitrogen, oxygen, and sulfur, and the sulfur may optionally be substituted with oxo (i.e., to form sulfoxide or sulfone), the remaining ring atoms being carbon. The bridged heterocyclyl is preferably 6- to 14-membered (e.g., 6-, 7-, 8-, 9-, 10-, 11-, 12-, 13-, and 14-membered) (i.e., 6- to 14-membered bridged heterocyclyl), and more preferably 7- to 10-membered (e.g., 7-, 8-, 9-, or 10-membered) (i.e., 7- to 10-membered bridged heterocyclyl). According to the number of the formed rings, the bridged heterocyclyl may be bicyclic or polycyclic (e.g., tricyclic or tetracyclic) bridged heterocyclyl, preferably bicyclic, tricyclic, or tetracyclic bridged heterocyclyl, and more preferably bicyclic or tricyclic bridged heterocyclyl. Non-limiting examples of bridged heterocyclyl include:

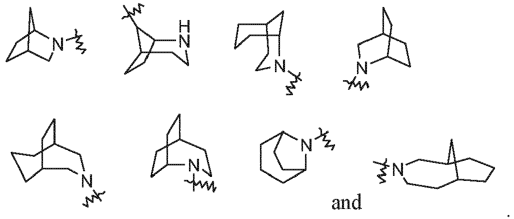

The heterocyclyl ring includes those in which the heterocyclyl described above (including monocyclic, spiro, fused, and bridged ones) is fused to an aryl, heteroaryl, or cycloalkyl ring, wherein the ring connected to the parent structure is heterocyclyl; its non-limiting examples include: 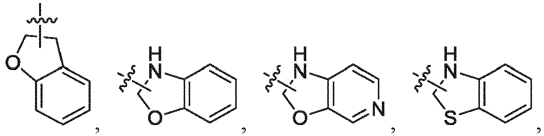 and the like.

Heterocyclyl may be substituted or unsubstituted. When substituted, it may be substituted at any available connection site, and the substituent is preferably selected from the group consisting of one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "aryl" refers to a 6- to 14-membered, preferably 6- to 10-membered all-carbon monocyclic or fused polycyclic (in which the rings share a pair of adjacent carbon atoms) group having a conjugated π-electron system, such as phenyl and naphthyl. The aryl ring includes those in which the aryl ring described above is fused to a heteroaryl, heterocyclyl, or cycloalkyl ring, wherein the ring connected to the parent structure is the aryl ring; its non-limiting examples include: 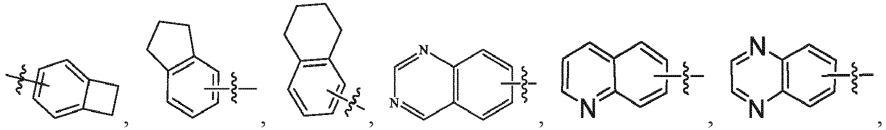 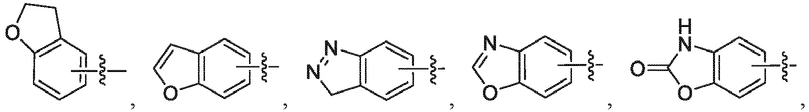 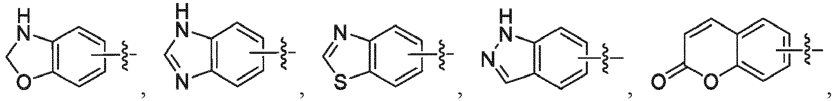 and

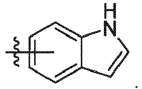

The aryl may be substituted or unsubstituted. When it is substituted, the substituent may be substituted at any available connection site, and the substituent is preferably selected from the group consisting of one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heteroaryl" refers to a heteroaromatic system containing 1 to 4 (e.g., 1, 2, 3, and 4) heteroatoms and 5 to 14 ring atoms, wherein the heteroatoms are selected from the group consisting of oxygen, sulfur, and nitrogen. The heteroaryl is preferably 5- to 10-membered (e.g., 5-, 6-, 7-, 8-, 9-, or 10-membered) (i.e., 5- to 10-membered heteroaryl), and more preferably 5-membered or 6-membered, e.g., furyl, thienyl, pyridinyl, pyrrolyl, *N*-alkylpyrrolyl, pyrimidinyl, pyrazinyl, pyridazinyl, imidazolyl, pyrazolyl, triazolyl, tetrazolyl, and the like. The heteroaryl ring includes those in which the heteroaryl described above is fused to an aryl, heterocyclyl, or cycloalkyl ring, wherein the ring connected to the parent structure is the heteroaryl ring; its non-limiting examples include:

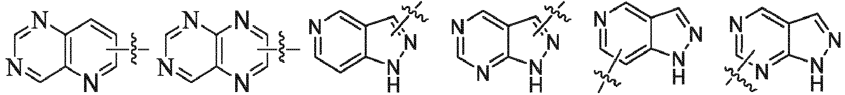

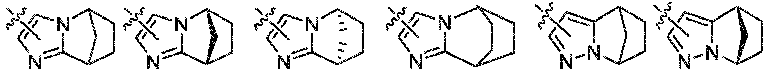

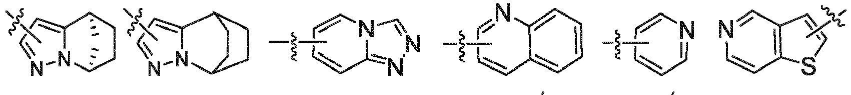

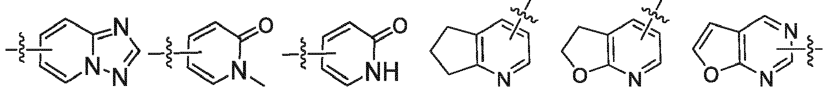

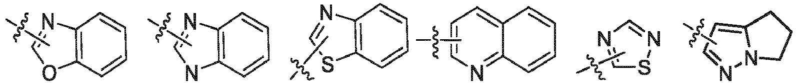

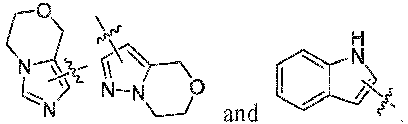

Heteroaryl may be substituted or unsubstituted. When substituted, it may be substituted at any available connection site, and the substituent is preferably selected from the group consisting of one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The cycloalkyl, heterocyclyl, aryl, and heteroaryl described above include residues derived from the parent ring by removal of one hydrogen atom from a ring atom, or residues derived from the parent ring by removal of two hydrogen atoms from the same ring atom or two different ring atoms, i.e., "divalent cycloalkyl", "divalent heterocyclyl", "arylene", and "heteroarylene".

The term "amino protecting group" refers to a group that can be easily removed and is intended to protect an amino group from being changed when reactions are taking place elsewhere in the molecule. Non-limiting examples include (trimethylsilyl)ethoxymethyl, tetrahydropyranyl, tert-butoxycarbonyl, acetyl, benzyl, allyl, *p*-methoxybenzyl, and the like. Those groups may be optionally substituted with 1 to 3 substituents selected from the group consisting of halogen, alkoxy, and nitro.

The term "hydroxy protecting group" refers to a hydroxy derivative that is commonly used to block or protect hydroxy while reactions are taking place on other functional groups of the compound. As an example, preferably, the hydroxyl protecting group may be triethylsilyl, triisopropylsilyl, *tert*-butyldimethylsilyl (TBS), *tert*-butyldiphenylsilyl, methyl, *tert*-butyl, benzyl, methoxymethyl (MOM), ethoxyethyl, formyl, acetyl, benzoyl, or *p*-nitrobenzoyl.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as defined above.

The term "halogen" refers to fluorine, chlorine, bromine, or iodine.

The term "hydroxy" refers to -OH.

The term "sulfhydryl" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O-, or (cycloalkyl)C(O)O-, wherein the alkyl and cycloalkyl are as defined above.

The compounds of the present disclosure may exist in specific stereoisomeric forms. The term "stereoisomer" refers to isomers that are structurally identical but differ in the arrangement of the atoms in space. It encompasses cis and trans (or *Z* and *E*) isomers, (-)- and (+)-isomers, (*R*)- and (*S*)-enantiomers, diastereomers, (*D*)- and (*L*)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. Optically active (-)- and (+)-isomers, (*R*)- and (*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. Furthermore, separation of enantiomers and diastereomers is generally accomplished by chromatography.

The compounds of the present disclosure may also be present in different tautomeric forms, and all such forms are included within the scope of the present disclosure. The term "tautomer" or "tautomeric form" refers to a structural isomer that exists in equilibrium and is readily converted from one isomeric form into another. It encompasses all possible tautomers; that is, it is present in the form of a single isomer or in the form of a mixture of the tautomers in any ratio. Non-limiting examples include: keto-enol, imine-enamine, lactam-lactim, and the like. An example of lactam-lactim in equilibrium is shown below:

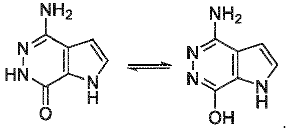

For example, reference to pyrazolyl is understood to include any one of the following two structures or a mixture of the two tautomers:

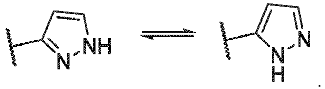

All tautomeric forms fall within the scope of the present disclosure, and the nomenclature of the compounds does not exclude any tautomers.

The compounds of the present disclosure include isotopic derivatives thereof. The term "isotopic derivative" refers to compounds that differ in structure only by having one or more enriched isotopic atoms. For example, compounds with the structure of the present disclosure having "deuterium" or "tritium" in place of hydrogen, or ¹⁸F-fluorine labeling (¹⁸F isotope) in place of fluorine, or ¹¹C-, ¹³C- or ¹⁴C-enriched carbon (¹¹C-, ¹³C- or ¹⁴C-carbon labeling; ¹¹C-, ¹³C- or ¹⁴C-isotope) in place of a carbon atom are within the scope of the present disclosure. Such a compound can be used as an analytical tool or a probe in, for example, a biological assay, or may be used as a tracer for *in vivo* diagnostic imaging of disease, or as a tracer in a pharmacodynamic, pharmacokinetic, or receptor study. The deuterated forms of the compound mean that each available hydrogen atom connected to a carbon atom may be independently replaced with a deuterium atom. Those skilled in the art can synthesize the compounds in deuterated form by reference to the relevant literature. Commercially available deuterated starting materials can be used in preparing the compounds in deuterated forms, or they can be synthesized using conventional techniques using deuterated reagents including, but not limited to, deuterated boranes, tri-deuterated boranes in tetrahydrofuran, deuterated lithium aluminum hydrides, deuterated iodoethanes, deuterated iodomethanes, and the like. Deuterides can generally retain comparable activity to non-deuterated compounds and can achieve better metabolic stability when deuterated at certain specific sites, thereby achieving certain therapeutic advantages. Compared with non-deuterated drugs, deuterated drugs have the advantages of reduced toxic and side effects, increased drug stability, enhanced curative effect, prolonged biological half-lives, and the like. All isotopic variations of the compounds of the present disclosure, whether radioactive or not, are intended to be included within the scope of the present disclosure. Each available hydrogen atom linked to a carbon atom may be independently replaced with a deuterium atom, wherein replacement of deuterium may be partial or complete, and replacement of partial deuterium refers to replacement of at least one hydrogen atom with at least one deuterium atom.

In the chemical structure of the compound of the present disclosure, a bond " 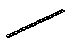 " represents an unspecified configuration; that is, if chiral isomers exist in the chemical structure, the bond " 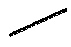 " may be " 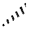 " or " 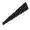 ", or contains both the configurations of " 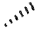 " and " 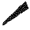 "**.**

"Optionally" or "optional" means that the event or circumstance subsequently described may, but does not necessarily, occur and that the description includes instances where the event or circumstance occurs or does not occur. For example, "C₁₋₆ alkyl that is optionally substituted with halogen or cyano" means that the halogen or cyano may, but does not necessarily, exist, and the description includes the instance where alkyl is substituted with halogen or cyano and the instance where alkyl is not substituted with halogen or cyano.

"Substituted" means that one or more, preferably 1-5, more preferably 1-3 hydrogen atoms in the group are independently substituted with a corresponding number of substituents. Those skilled in the art can determine (experimentally or theoretically) possible or impossible substitution without undue effort. For example, it may be unstable when amino or hydroxy having free hydrogen is bound to a carbon atom having an unsaturated (e.g., olefinic) bond.

The term "pharmaceutical composition" refers to a mixture containing one or more of the compounds described herein or a pharmaceutically acceptable salt or pro-drug thereof, and other chemical components, and other components, for example, pharmaceutically acceptable carriers and excipients. The pharmaceutical composition is intended to promote the administration to an organism, so as to facilitate the absorption of the active ingredient, thereby exerting biological activity.

"Pharmaceutically acceptable salt" refers to a salt of the compound of the present disclosure, which may be selected from the group consisting of inorganic and organic salts. The salts are safe and effective for use in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount" refers to an amount of the drug or agent sufficient to achieve, or at least partially achieve, the desired effect. The determination of the effective amount varies from person to person. It depends on the age and general condition of a subject, as well as the particular active substance used. The appropriate effective amount in a case may be determined by those skilled in the art in the light of routine tests.

The term "pharmaceutically acceptable" used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an", and "the" include plural references and vice versa, unless otherwise clearly defined in the context.

When the term "about" is applied to parameters such as pH, concentration and temperature, it means that the parameter may vary by ±10%, and sometimes more preferably within ±5%. As will be appreciated by those skilled in the art, when the parameters are not critical, the numbers are generally given for illustrative purposes only and are not intended to be limiting.

### Synthetic methods of the compounds of the present disclosure

To achieve the purpose of the present disclosure, the following technical schemes are adopted in the present disclosure:

### Scheme 1

A method for preparing the compound of general formula (IM) or the pharmaceutically acceptable salt thereof of the present disclosure is provided, and the method comprises the following step: 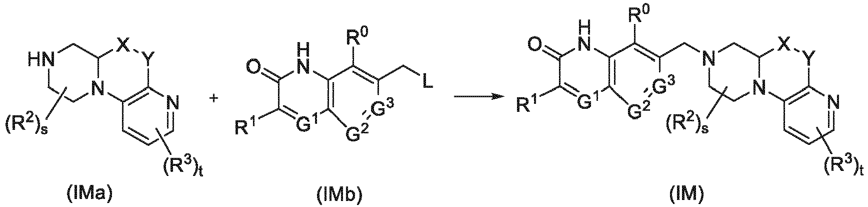
conducting a nucleophilic substitution reaction of a compound of general formula (IMa) or a salt (preferably hydrochloride) thereof with a compound of general formula (IMb) under alkaline conditions, optionally in the presence of a catalyst, to give the compound of general formula (IM) or the pharmaceutically acceptable salt thereof;
wherein:
   L is halogen, preferably a chlorine atom;
   X, Y, G¹ to G³, R⁰ to R³, s, and t are as defined in general formula (IM).

### Scheme 2

A method for preparing the compound of general formula (I) or the pharmaceutically acceptable salt thereof of the present disclosure is provided, and the method comprises the following steps: 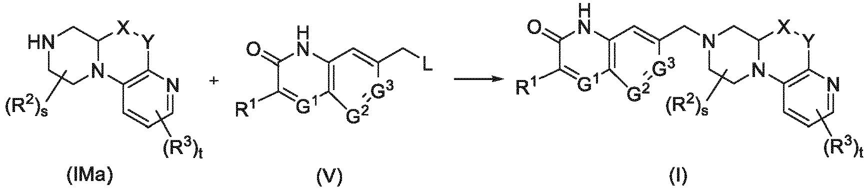
conducting a nucleophilic substitution reaction of a compound of general formula (IMa) or a salt (preferably hydrochloride) thereof with a compound of general formula (V) under alkaline conditions, optionally in the presence of a catalyst, to give the compound of general formula (I) or the pharmaceutically acceptable salt thereof;
wherein:
   L is halogen, preferably a chlorine atom;
   X, Y, G¹ to G³, R¹ to R³, s, and t are as defined in general formula (I).

### Scheme 3

A method for preparing the compound of general formula (II-1) or the pharmaceutically acceptable salt thereof of the present disclosure is provided, and the method comprises the following step: 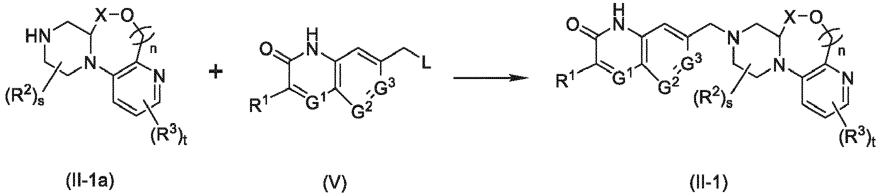
conducting a nucleophilic substitution reaction of a compound of general formula (II-1a) or a salt (preferably hydrochloride) thereof with a compound of general formula (V) under alkaline conditions, optionally in the presence of a catalyst, to give the compound of general formula (II-1) or the pharmaceutically acceptable salt thereof;
wherein:
   L is halogen, preferably a chlorine atom;
   X, G¹ to G³, R¹ to R³, s, t, and n are as defined in general formula (II-1).

### Scheme 4

A method for preparing the compound of general formula (II-2) or the pharmaceutically acceptable salt thereof of the present disclosure is provided, and the method comprises the following step: 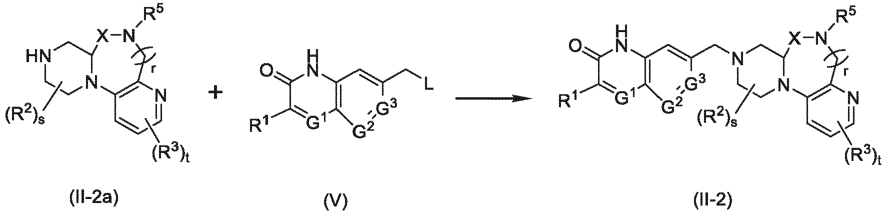
conducting a nucleophilic substitution reaction of a compound of general formula (II-2a) or a salt (preferably hydrochloride) thereof with a compound of general formula (V) under alkaline conditions, optionally in the presence of a catalyst, to give the compound of general formula (II-2) or the pharmaceutically acceptable salt thereof;
wherein:
   L is halogen, preferably a chlorine atom;
   X, G¹ to G³, R¹ to R³, R⁵, s, t, and r are as defined in general formula (II-2).

### Scheme 5

A method for preparing the compound of general formula (III-1) or the pharmaceutically acceptable salt thereof of the present disclosure is provided, and the method comprises the following step: 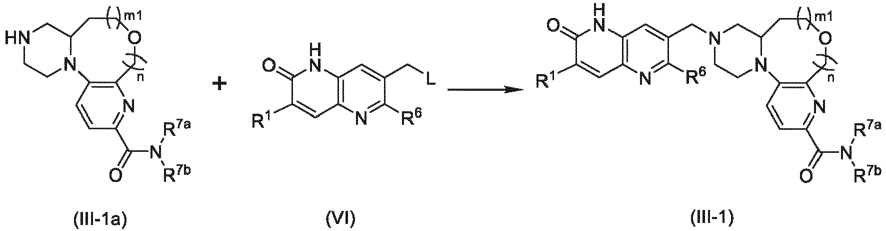
conducting a nucleophilic substitution reaction of a compound of general formula (III-1a) or a salt (preferably hydrochloride) thereof with a compound of general formula (VI) under alkaline conditions, optionally in the presence of a catalyst, to give the compound of general formula (III-1) or the pharmaceutically acceptable salt thereof;
wherein:
   m1 is 0 or 1;
   L is halogen, preferably a chlorine atom;
   R¹, R⁶, R^{7a}, R^{7b}, and n are as defined in general formula (III-1).

### Scheme 6

A method for preparing the compound of general formula (III-1-A) or the pharmaceutically acceptable salt thereof of the present disclosure is provided, and the method comprises the following step: 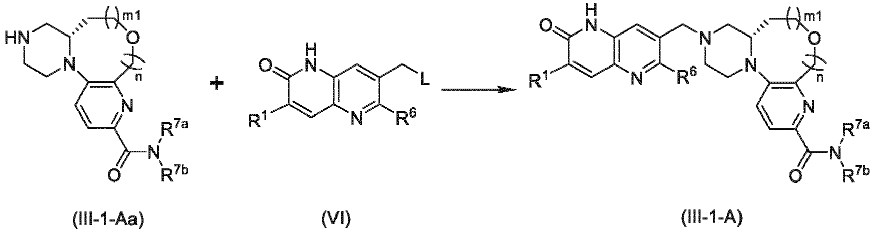
conducting a nucleophilic substitution reaction of a compound of general formula (III-1-Aa) or a salt (preferably hydrochloride) thereof with a compound of general formula (VI) under alkaline conditions, optionally in the presence of a catalyst, to give the compound of general formula (III-1-A) or the pharmaceutically acceptable salt thereof;
wherein:
   m1 is 0 or 1;
   L is halogen, preferably a chlorine atom;
   R¹, R⁶, R^{7a}, R^{7b}, and n are as defined in general formula (III-1-A).

### Scheme 7

A method for preparing the compound of general formula (III-2) or the pharmaceutically acceptable salt thereof of the present disclosure is provided, and the method comprises the following step: 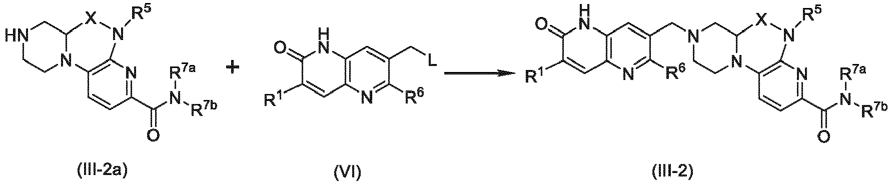
conducting a nucleophilic substitution reaction of a compound of general formula (III-2a) or a salt (preferably hydrochloride) thereof with a compound of general formula (VI) under alkaline conditions, optionally in the presence of a catalyst, to give the compound of general formula (III-2) or the pharmaceutically acceptable salt thereof;
wherein:
   L is halogen, preferably a chlorine atom;
   X, R¹, R⁵, R⁶, R^{7a}, and R^{7b} are as defined in general formula (III-2).

Reagents that provide alkaline conditions include organic bases and inorganic bases. The organic bases include, but are not limited to, triethylamine, *N,N-*diisopropylethylamine, *n*-butyllithium, lithium diisopropylamide, sodium acetate, potassium acetate, sodium ethoxide, sodium *tert*-butoxide, and potassium *tert*-butoxide, preferably *N,N-*diisopropylethylamine. The inorganic bases include, but are not limited to, sodium hydride, potassium phosphate, sodium carbonate, potassium carbonate, cesium carbonate, sodium hydroxide, lithium hydroxide monohydrate, lithium hydroxide, and potassium hydroxide.

The catalyst for the nucleophilic substitution reaction described above is sodium iodide or potassium iodide, preferably sodium iodide.

The reactions described above are preferably conducted in solvents including but not limited to *N*-methylpyrrolidone, ethylene glycol dimethyl ether, acetic acid, methanol, ethanol, acetonitrile, *n*-butanol, toluene, tetrahydrofuran, dichloromethane, petroleum ether, ethyl acetate, *n*-hexane, dimethyl sulfoxide, 1,4-dioxane, water, *N*,*N*-dimethylformamide, *N,N-*dimethylacetamide, 1,2-dibromoethane, and mixtures thereof.

### DETAILED DESCRIPTION

The present disclosure is further described below with reference to examples, but these examples are not intended to limit the scope of the present disclosure.

### Examples

The structures of compounds were determined by nuclear magnetic resonance (NMR) spectroscopy and/or mass spectrometry (MS). NMR shifts (δ) are given in 10⁻⁶ (ppm). NMR spectra were determined using a Bruker AVANCE-400 nuclear magnetic resonance instrument or Bruker AVANCE NEO 500M, with deuterated dimethyl sulfoxide (DMSO-*d₆*), deuterated chloroform (CDCl₃), and deuterated methanol (CD₃OD) as determination solvents and tetramethylsilane (TMS) as an internal standard.

Mass spectrometry (MS) analyses were performed on an Agilent 1200/1290 DAD-6110/6120 Quadrupole MS liquid chromatography-mass spectrometry system (manufacturer: Agilent; MS model: 6110/6120 Quadrupole MS),
waters ACQuity UPLC-QD/SQD (manufacturer: waters; MS model: waters ACQuity Qda Detector/waters SQ Detector), and THERMO Ultimate 3000-Q Exactive (manufacturer: THERMO; MS model: THERMO Q Exactive).

High performance liquid chromatography (HPLC) analyses were performed on Agilent HPLC 1200DAD, Agilent HPLC 1200VWD, and Waters HPLC e2695-2489 liquid chromatographs.

Chiral HPLC analyses were performed on an Agilent 1260 DAD high performance liquid chromatograph.

Preparative high performance liquid chromatography used Waters 2545-2767, Waters 2767-SQ Detecor2, Shimadzu LC-20AP, and Gilson GX-281 preparative chromatographs.

Preparative chiral chromatography used a Shimadzu LC-20AP preparative chromatograph.

The CombiFlash preparative flash chromatograph used was Combiflash Rf200 (TELEDYNE ISCO).

Yantai Huanghai HSGF254 or Qingdao GF254 silica gel plates, 0.15-0.2 mm layer thickness, were adopted for thin-layer chromatography (TLC) analysis and 0.4-0.5 mm layer thickness for TLC separation and purification.

Silica gel column chromatography generally used 200- to 300-mesh silica gel (Huanghai, Yantai) as the carrier.

The mean inhibition of kinase and the IC₅₀ value were measured on a NovoStar microplate reader (BMG, Germany).

Known starting materials in the present disclosure may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Pressurized hydrogenation reactions were performed using a Parr 3916EKX hydrogenator and a Qinglan QL-500 hydrogenator, or an HC2-SS hydrogenator. Hydrogenation reactions generally involved 3 cycles of vacuumization and hydrogen purging.

Microwave reactions were performed on a CEM Discover-S 908860 microwave reactor. In the examples, a solution was an aqueous solution unless otherwise specified.

In the examples, reactions were conducted at room temperature, i.e., 20 °C to 30 °C, unless otherwise specified.

The monitoring of the reaction progress in the examples was conducted by thin-layer chromatography (TLC). The developing solvent for reactions, the eluent system for column chromatography purification, and the developing solvent system for thin-layer chromatography include: A: dichloromethane/methanol system. The volume ratio of the solvents was adjusted according to the polarity of the compound, or by adding a small amount of basic or acidic reagents such as triethylamine and acetic acid.

### Example 1

### (±)-3-((7-Ethyl-6-oxo-5,6-dihydro-1,5-naphthyridin-3-yl)methyl)-N-methyl-1,2,3,4,4a,5 -hexahydropyrazino[1,2-d]pyrido[2,3-b][1,4]oxazine-8-carboxamide 1

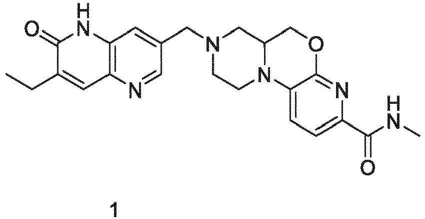

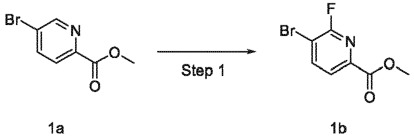

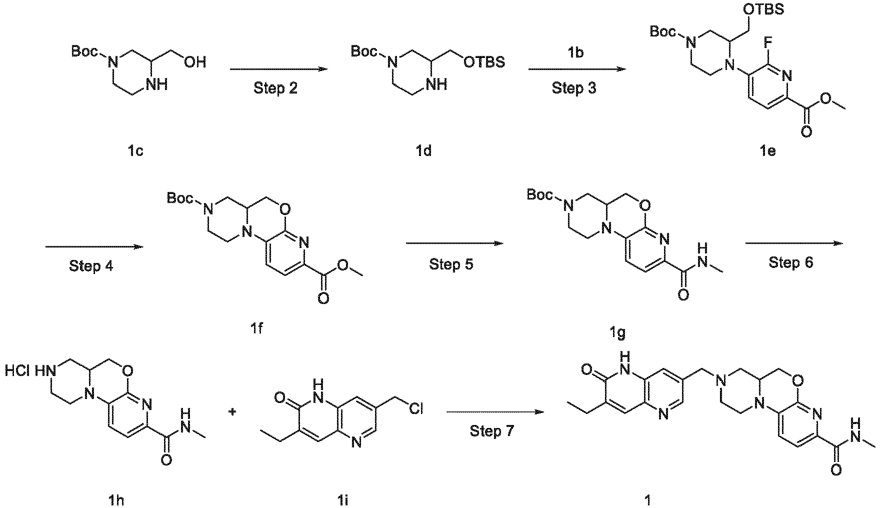

### Step 1

### Methyl 5-bromo-6-fluoropicolinate 1b

Compound methyl 5-bromopyridine-2-carboxylate **1a** (2.0 g, 9.25 mmol, Shanghai Accela ChemBio Co., Ltd.) was dissolved in acetonitrile (50 mL), silver difluoride (4.7 g, 32.22 mmol) was added, and the mixture was stirred in a nitrogen atmosphere for 14 h. The reaction mixture was filtered and concentrated under reduced pressure. The residue was purified by silica gel column chromatography using eluent system B to give the title compound **1b** (1.8 g, 83% yield).

MS m/z (ESI): 233.9 [M+1].

### Step 2

### tert-Butyl (±)-3-(((tert-butyldimethylsilyl)oxy)methyl)piperazine-1-carboxylate 1d

Compound *tert*-butyl (±)-3-(hydroxymethyl)piperazine-1-carboxylate **1c** (2 g, 9.24 mmol, Shanghai Bide Pharmatech Ltd.) was dissolved in dichloromethane (30 mL), imidazole (1.3 g, 19.09 mmol) and *tert*-butyldimethylchlorosilane (2.17 g, 14.39 mmol) were added, and the mixture was stirred for 1 h. The reaction mixture was concentrated under reduced pressure. The residue was purified by silica gel column chromatography using eluent system A to give the title compound **1d** (1.9 g, 62.1% yield).

MS m/z (ESI): 331.2 [M+1].

### Step 3

### tert-Butyl (±)-3-(((tert-butyl dimethyl silyl)oxy)methyl)-4-(2-fluoro-6-(methoxycarbonyl)pyri din-3-yl)piperazine-1-carboxylate 1e

Compound **1b** (300 mg, 1.28 mmol) and compound **1d** (635 mg, 1.92 mmol) were dissolved in 1,4-dioxane (15 mL), methanesulfonato(2-dicyclohexylphosphino-2',6'-diisopropoxy-1,1'-biphenyl)(2-amino-1,1'-biphenyl-2-yl)palladium(II) (107 mg, 127.78 µmol) and cesium carbonate (1.0 g, 3.06 mmol) were added, and the mixture was reacted in a nitrogen atmosphere at 110 °C for 14 h. The reaction mixture was concentrated under reduced pressure. The residue was purified by silica gel column chromatography using eluent system A to give the title compound **1e** (290 mg, 46.7% yield).

MS m/z (ESI): 484.2 [M+1].

### Step 4

### (±)-3-(tert-Butyl) 8-methyl-1,2,4a,5-tetrahydropyrazino[1,2-d]pyrido[2,3-b][1,4]oxazine-3,8(4H)-dicarbo xylate 1f

Compound **1e** (290 mg, 599.61 µmol) was dissolved in tetrahydrofuran (6 mL), a solution of tetrabutylammonium fluoride in tetrahydrofuran (3 mL, 1 M) was added, and the mixture was stirred for 2 h. The reaction mixture was concentrated under reduced pressure to give crude title compound **1f** (200 mg, 95.2% yield), which was directly used in the next reaction without purification.

MS m/z (ESI): 350.2 [M+1].

### Step 5

### tert-Butyl (±)-8-(methylaminocarbonyl)-1,2,4a,5-tetrahydropyrazino[1,2-d]pyrido[2,3-b][1,4]oxaz ine-3(4H)-carboxylate 1g

The crude compound **1f** (200 mg, 572.44 µmol) was dissolved in ethanol (5 mL), a solution of methylamine in ethanol (1.13 mL, 1 M) was added, and the mixture was stirred for 14 h. The reaction mixture was concentrated under reduced pressure. The residue was purified by silica gel column chromatography using eluent system A to give the title compound **1g** (190 mg, 95.4% yield).

MS m/z (ESI): 349.2 [M+1].

### Step 6

### (±)-N-Methyl-1,2,3,4,4a,5-hexahydropyrazino[1,2-d]pyrido[2,3-b][1,4]oxazine-8-carbo xamide hydrochloride 1h

Compound **1g** (50 mg, 143.5 µmol) was dissolved in dioxane (2 mL), a solution of hydrochloric acid in dioxane (1 mL, 4 M) was added, and the mixture was stirred for 0.5 h. The reaction mixture was concentrated under reduced pressure to give crude title compound **1h** (35 mg, 98.2% yield), which was directly used in the next reaction without purification.

MS m/z (ESI): 249.2 [M+1].

### Step 7

### (±)-3-((7-Ethyl-6-oxo-5,6-dihydro-1,5-naphthyridin-3-yl)methyl)-N-methy1-1,2,3,4,4a,5 -hexahydropyrazino[1,2-d]pyrido[2,3-b][1,4]oxazine-8-carboxamide 1

The crude compound **1h** (30 mg, 134.72 µmol), compound 7-(chloromethyl)-3-ethyl-1,5-naphthyridin-2(1*H*)-one **1i** (33 mg, 132.91 µmol, prepared by the method disclosed in Example 4 on page 15 of the specification in the patent application "WO2021013735A1"), *N*,*N*-diisopropylethylamine (174 mg, 1.34 mmol), and sodium iodide (4 mg, 26.68 µmol) were dissolved in acetonitrile (3 mL), and the mixture was reacted at 80 °C for 3 h. The reaction mixture was concentrated under reduced pressure. The crude product was purified by high performance liquid chromatography (Waters-2545, column: SharpSil-T C18, 30 mm × 150 mm, 5 µm; mobile phase: aqueous phase (10 mmol/L ammonium bicarbonate) and acetonitrile; gradient ratio: acetonitrile 35%-45%; flow rate: 30 mL/min) to give the title compound **1** (4 mg, 6.8% yield).

MS m/z (ESI): 435.2 [M+1].

¹H NMR (500 MHz, CDCl₃): δ 10.11 (s, 1H), 8.53 (d, 1H), 7.86 (s, 1H), 7.81 (d, 1H), 7.61 (d, 1H), 7.58-7.54 (m, 1H), 7.10 (d, 1H), 4.37 (dd, 1H), 4.17 (dd, 1H), 3.76 (d, 1H), 3.73-3.65 (m, 2H), 3.40-3.30 (m, 1H), 3.09-3.02 (m, 1H), 2.99 (d, 2H), 2.89 (dt, 1H), 2.80-2.68 (m, 2H), 2.41 (td, 1H), 2.25 (t, 1H), 2.03 (d, 1H), 1.98 (t, 1H), 1.33 (t, 3H).

### Example 2

### (R)-3-((7-Ethyl-6-oxo-5,6-dihydro-1,5-naphthyridin-3-yl)methyl)-N-methyl-1,2,3,4,4a,5 -hexahydropyrazino[1,2-d]pyrido[2,3-b][1,4]oxazine-8-carboxamide 2

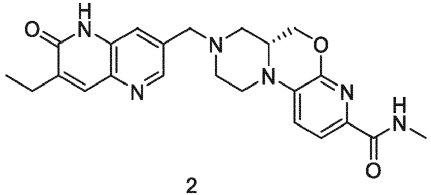

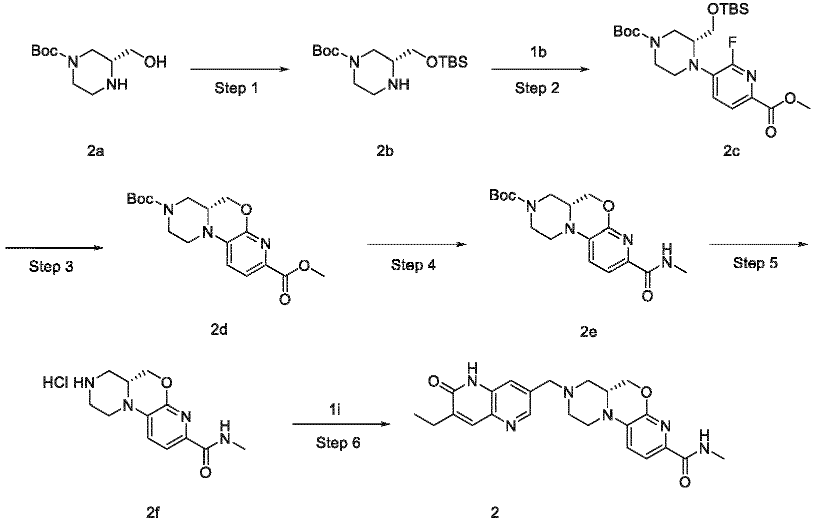

### Step 1

*tert*-Butyl (*R*)-3-(((*tert*-butyldimethylsilyl)oxy)methyl)piperazine-1-carboxylate **2b** Compound *tert*-butyl (3*R*)-3-(hydroxymethyl)piperazine-1-carboxylate **2a** (4.2 g, 19.4 mmol, Shanghai Bide Pharmatech Ltd.) was dissolved in dichloromethane (100 mL), triethylamine (117 mg, 38.9 mmol), tert-butyldimethylchlorosilane (2.17 g, 14.39 mmol), and 4-dimethylaminopyridine (3.94 g, 0.94 mmol) were added, and the mixture was stirred for 14 h. The reaction mixture was concentrated under reduced pressure. The residue was purified by silica gel column chromatography using eluent system A to give the title compound **2b** (6 g, 93% yield).

MS m/z (ESI): 331.2 [M+1].

### Step 2

### tert-Butyl (R)-3-(((tert-butyldimethylsilyl)oxy)methyl)-4-(2-fluoro-6-(methoxycarbonyl)pyridin-3 -yl)piperazine-1-carboxylate 2c

Compound **1b** (1.3 g, 5.55 mmol) and compound **2b** (2.02 g, 6.1 mmol) were dissolved in 1,4-dioxane (15 mL), methanesulfonato(2-dicyclohexylphosphino-2',6'-diisopropoxy-1,1'-biphenyl)(2-amino-1,1'-biphenyl-2-yl)palladium(II) (464 mg, 554.7 µmol) and cesium carbonate (3.6 g, 11.1 mmol) were added, and the mixture was reacted in a nitrogen atmosphere at 110 °C for 14 h. The reaction mixture was concentrated under reduced pressure. The residue was purified by silica gel column chromatography using eluent system A to give the title compound **2c** (1 g, 37.2% yield).

MS m/z (ESI): 484.2 [M+1].

### Step 3

### 3 -(tert-Butyl) 8-methyl-(R)-1,2,4a,5-tetrahydropyrazino[1,2-d]pyrido[2,3-b][1,4]oxazine-3,8(4H)-dica rboxylate 2d

Compound **2c** (1 g, 2.06 mmol) was dissolved in tetrahydrofuran (6 mL), a solution of tetrabutylammonium fluoride in tetrahydrofuran (6 mL, 1 M) was added, and the mixture was stirred for 2 h. The reaction mixture was concentrated under reduced pressure to give crude title compound **2d** (722 mg, 99% yield), which was directly used in the next reaction without purification.

MS m/z (ESI): 350.2 [M+1].

### Step 4

### tert-Butyl (R)-8-(methylaminocarbonyl)-1,2,4a,5-tetrahydropyrazino[1,2-d]pyrido[2,3-b][1,4]oxaz ine-3(4H)-carboxylate 2e

The crude compound **2d** (722 mg, 2.06 mmol) was dissolved in a solution of methylamine in ethanol (10 mL, 1 M), and the mixture was stirred for 2 h. The reaction mixture was concentrated under reduced pressure to give crude title compound **2e** (700 mg, 97% yield), which was directly used in the next reaction without purification.

MS m/z (ESI): 349.2 [M+1].

### Step 5

### (R)-N-Methyl-1,2,3,4,4a,5-hexahydropyrazino[1,2-d]pyrido[2,3-b][1,4]oxazine-8-carbo xamide hydrochloride 2f

Compound **2e** (700 mg, 2 mmol) was dissolved in dichloromethane (5 mL), a solution of hydrochloric acid in dioxane (2 mL, 4 M) was added, and the mixture was stirred for 0.5 h. The reaction mixture was concentrated under reduced pressure to give crude title compound **2f** (570 mg, 99% yield), which was directly used in the next reaction without purification.

MS m/z (ESI): 249.2 [M+1].

### Step 6

### (R)-3-((7-Ethyl-6-oxo-5,6-dihydro-1,5-naphthyridin-3-yl)methyl)-N-methyl-1,2,3,4,4a,5 -hexahydropyrazino[1,2-d]pyrido[2,3-b][1,4]oxazine-8-carboxamide 2

The crude compound **2f** (66 mg, 265.82 µmol), compound **1i** (60 mg, 269.4 µmol), and *N,N*-diisopropylethylamine (180.7 mg, 1.4 mmol) were dissolved in acetonitrile (5 mL), a catalytic amount of sodium iodide was added, and the mixture was reacted at 80 °C for 5 h. The reaction mixture was concentrated under reduced pressure. The crude product was purified by high performance liquid chromatography (Waters-2545, column: SharpSil-T C18, 30 mm × 150 mm, 5 µm; mobile phase: aqueous phase (10 mmol/L ammonium bicarbonate) and acetonitrile; gradient ratio: acetonitrile 35%-45%; flow rate: 30 mL/min) to give the title compound **2** (33 mg, 28.1% yield).

MS m/z (ESI): 435.2 [M+1].

¹H NMR (500 MHz, CD₃OD): δ 8.48 (d,1H), 7.82 (s, 1H), 7.76 (d,1H), 7.61 (d,1H), 7.23 (d,1H), 4.39 (dd,1H), 4.11 (dd,1H), 3.84-3.67 (m, 3H), 3.26 (d, 1H), 3.04 (d,1H), 2.95 (dt,1H), 2.89 (d, 4H), 2.66 (q,2H), 2.41-2.33 (m, 1H), 1.96 (t,1H), 1.27 (t,3H).

### Example 3

### (±)-3-((7-Ethyl-6-oxo-5,6-dihydro-1,5-naphthyridin-3-yl)methyl)-N-methyl-1,2,3,4,4a,5 -hexahydro-7H-pyrazino[2,1-c]pyrido[3,2-e][1,4]oxazepine-9-carboxamide 3

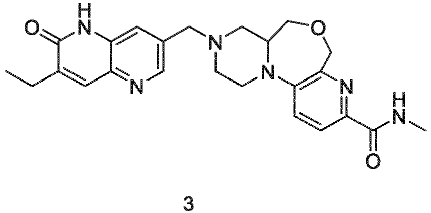

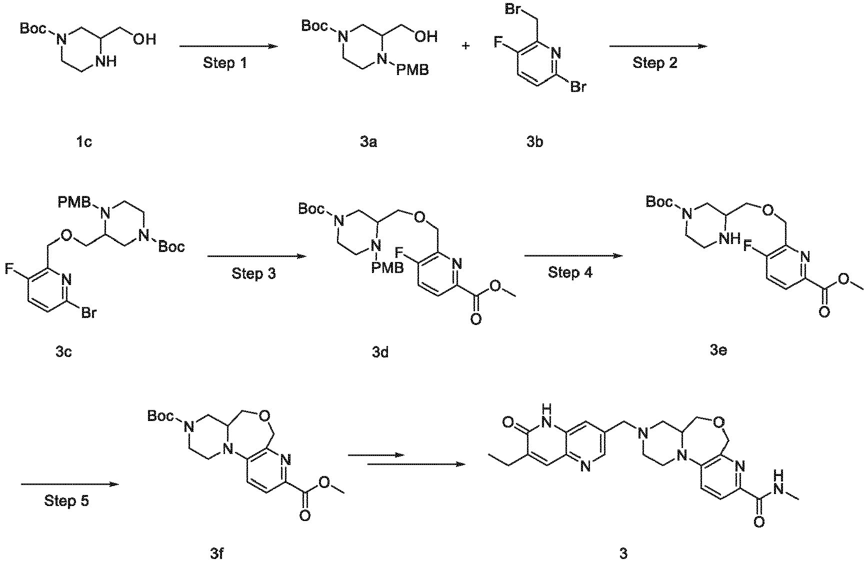

### Step 1

### tert-Butyl (±)-3-(hydroxymethyl)-4-(4-methoxybenzyl)piperazine-1-carboxylate 3a

Compound **1c** (5 g, 23.1 mmol) was dissolved in *N*,*N*-dimethylformamide (50 mL), anhydrous potassium carbonate (4.8 g, 34.7 mmol) and 4-methoxybenzyl chloride (4 g, 25.54 mmol) were added, and the mixture was reacted at 60 °C for 72 h. The reaction mixture was filtered and concentrated under reduced pressure. The residue was purified by silica gel column chromatography using eluent system B to give the title compound **3a** (7 g, 90% yield).

MS m/z (ESI): 337.2 [M+1].

### Step 2

*tert*-Butyl

### (±)-3-(((6-bromo-3-fluoropyridin-2-yl)methoxy)methyl)-4-(4-methoxybenzyl)piperazin e-1-carboxylate 3c

Compound 6-bromo-2-(bromomethyl)-3-fluoropyridine **3b** (1.94 g, 7.21 mmol, prepared by the method disclosed in Preparation Example 6 on page 12 of the specification in the patent application "WO2016077161A1") and compound **3a** (2.2 g, 6.59 mmol) were dissolved in *N,N*-dimethylformamide (20 mL), sodium hydride (0.36 g, 9 mmol, 60% purity) was added, and the mixture was stirred for 1 h. Water was added to quench the reaction. The reaction mixture was extracted with dichloromethane (30 mL × 3). The organic phases were combined, dried over anhydrous sodium sulfate, and filtered to remove the desiccant, and then the filtrate was concentrated under reduced pressure. The residue was purified by silica gel column chromatography using eluent system B to give the title compound **3c** (1.84 g, 53.6% yield).

MS m/z (ESI): 524.2 [M+1].

### Step 3

### tert-Butyl (±)-3-((3-fluoro-6-(methoxycarbonyl)pyridin-2-yl)methoxy)methyl)-4-(4-methoxybenz yl)piperazine-1-carboxylate 3d

Compound **3c** (1.84 g, 3.50 mmol) was dissolved in a mixed solvent of *N,N*-dimethylformamide (3 mL) and ethanol (2 mL), bis(triphenylphosphine)palladium(II) dichloride (0.55 g, 783.59 µmol) and *N,N-*diisopropylethylamine (1.1 g, 10.87 mmol) were added, and the mixture was stirred in a carbon monoxide atmosphere at 90 °C for 3 h. The reaction mixture was cooled, diluted by adding ethyl acetate (100 mL), and washed sequentially with water and a saturated sodium chloride solution. The organic phase was collected, dried over anhydrous sodium sulfate, and filtered to remove the desiccant, and then the filtrate was concentrated under reduced pressure. The residue was purified by silica gel column chromatography using eluent system B to give the title compound **3d** (1.1 g, 60.57% yield).

MS m/z (ESI): 504.2 [M+1].

### Step 4

### tert-Butyl (±)-3-(((3-fluoro-6-(methoxycarbonyl)pyridin-2-yl)methoxy)methyl)piperazine-1-carbo xylate 3e

Compound **3d** (0.4 g, 772.81 µmol) was dissolved in a mixed solvent of water (3 mL) and acetonitrile (3 mL), ceric ammonium nitrate (2.11 g, 3.86 mmol) was added, and the mixture was purged with hydrogen and stirred for 14 h. A saturated aqueous sodium bicarbonate solution (10 mL) was added for neutralization. The reaction mixture was filtered, and the filtrate was extracted with ethyl acetate (10 mL × 3). The organic phase was collected, washed with saturated brine, dried over anhydrous sodium sulfate, and filtered to remove the desiccant, and then the filtrate was concentrated under reduced pressure to give crude title compound **3e** (120 mg, 40% yield).

MS m/z (ESI): 384.2 [M+1].

### Step 5

### (±)-3-(tert-Butyl) 9-methyl-1,2,4a,5-tetrahydro-7H-pyrazino[2,1-c]pyrido[3,2-e][1,4]oxazepine-3,9(4H)-d icarboxylate 3f

Compound **3e** (300 mg, 754.8 µmol) was dissolved in *N*,*N*-dimethylacetamide (2 mL), *N,N*-diisopropylethylamine (300 mg, 2.32 mmol) was added, and the mixture was reacted under microwave at 140 °C for 1.5 h. The reaction mixture was cooled and concentrated under reduced pressure. The residue was purified by silica gel column chromatography using eluent system A to give the title compound **3f** (130 mg, 45.6% yield).

MS m/z (ESI): 364.2 [M+1].

Subsequently, the title compound **3** (7.5 mg, 14.6% yield) was obtained by following the synthetic route of Example 1 with the starting material compound **1f** in step 5 replaced by compound **3f.**

MS m/z (ESI): 449.2 [M+1].

1H NMR (500 MHz, CD₃OD): δ 8.51 (d, 1H), 7.93 (d, 1H), 7.86 (s, 1H), 7.79 (d, 1H), 7.50 (d, 1H), 4.99 (t, 2H), 4.85 (d, 1H), 4.08 (dd, 2H), 3.87 (dd, 1H), 3.81-3.71 (m, 2H), 3.46 (ddd, 2H), 2.95 (s, 3H), 2.86- 2.78 (m, 1H), 2.75-2.61 (m, 3H), 2.57 (dd, 1H), 1.31 (t, 3H).

### Example 4

### (±)-3-((7-Ethyl-6-oxo-5,6-dihydro-1,5-naphthyridin-3-yl)methyl)-N-methyl-2,3,4,4a,5,6 -hexahydro- 1H-pyrazino[1,2-d]pyrido[2,3-b][1,4]oxazepine-9-carboxamide 4

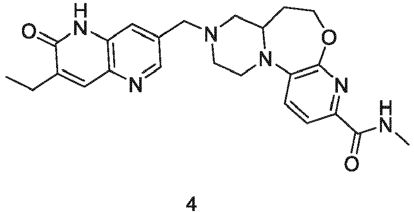

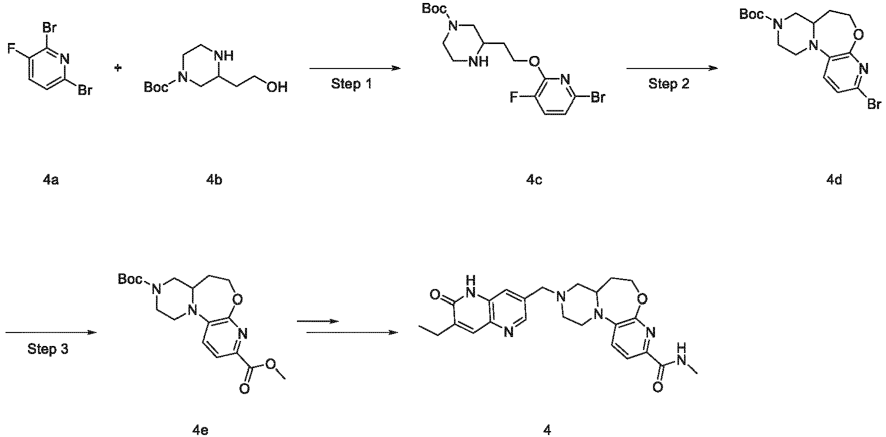

### Step 1

### tert-Butyl (±)-3-(2-((6-bromo-3-fluoropyridin-2-yl)oxy)ethyl)piperazine-1-carboxylate 4c

Compound 2,6-dibromo-3-fluoropyridine **4a** (1.0 g, 3.92 mmol, Shanghai Bide Pharmatech Ltd.) and *tert*-butyl (±)-3-(2-hydroxyethyl)piperazine-1-carboxylate **4b** (900 mg, 3.90 mmol, Jiangsu Aikon) were dissolved in tetrahydrofuran (20 mL), potassium *tert*-butoxide (880 mg, 7.84 mmol) was added, and the mixture was stirred for 2 h. The reaction mixture was concentrated under reduced pressure. The residue was purified by silica gel column chromatography using eluent system A to give the title compound **4c** (1 g, 63% yield).

MS m/z (ESI): 404.2 [M+1].

### Step 2

### tert-Butyl (±)-9-bromo-1,2,4,4a,5,6-hexahydro-3H-pyrazino[1,2-d]pyrido[2,3-b][1,4]oxazepine-3-carboxylate 4d

Compound **4c** (40 mg, 98.9 µmol) was dissolved in *N,N*-dimethylacetamide (2 mL), *N,N-*diisopropylethylamine (38 mg, 294 µmol) was added, and the mixture was reacted under microwave at 140 °C for 2 h. The reaction mixture was cooled and concentrated under reduced pressure to give crude title compound **4d** (38 mg, 99% yield), which could be used in the next reaction without purification.

MS m/z (ESI): 384.2 [M+1].

### Step 3

### (±)-3 -(tert-Butyl) 9-methyl-1,2,4,4a,5,6-hexahydro-3H-pyrazino[1,2-d]pyrido[2,3-b][1,4]oxazepine-3,9-di carboxylate 4e

The crude compound **4d** (100 mg, 247.3 µmol) was dissolved in a mixed solvent of *N*,*N*-dimethylacetamide (2 mL) and methanol (5 mL), palladium acetate (17 mg, 75.72 µmol), triethylamine (125 mg, 1.23 mmol), and 1,3-bis(diphenylphosphino)propane (30 mg, 72.72 µmol) were added, and the mixture was reacted in a carbon monoxide atmosphere at 80 °C for 14 h. The reaction mixture was cooled and concentrated under reduced pressure. The residue was purified by silica gel column chromatography using eluent system A to give the title compound **4e** (40 mg, 42.1% yield).

MS m/z (ESI): 364.2 [M+1].

Subsequently, the title compound **4** (130 mg, 52.1% yield) was obtained by following the synthetic route of Example 1 with the starting material compound **1f** in step 5 replaced by compound **4e.**

MS m/z (ESI): 449.2 [M+1].

1H NMR (500 MHz, CDCl₃): δ 11.38 (s, 1H), 8.57 (d, 1H), 7.90 (q, 1H), 7.70 (d, 1H), 7.68 (d, 1H), 7.68-7.62 (m, 1H), 7.19 (d, 1H), 4.46 (ddd, 1H), 4.28 (ddd, 1H),3.73 (s, 2H), 3.68 (dd, 1H), 3.68-3.52 (m, 2H), 3.02 (d, 3H), 2.89-2.82 (m, 1H), 2.78 (qd, 2H), 2.68 (ddd, 1H), 2.57 (ddd, 1H), 2.34 (dd, 1H), 2.21-2.00 (m, 2H), 1.35 (t, 3H).

### Example 5

### (±)-3-((7-Ethyl-6-oxo-5,6-dihydro-1,5-naphthyridin-3-yl)methyl)-N-methyl-5-oxo-2,3,4 ,4a,5,6-hexahydro-1H-pyrazino[1,2-a]pyrido[2,3-e]pyrazine-S-carboxamide 5

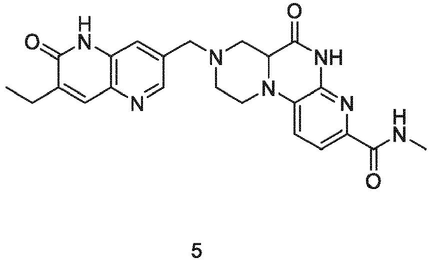

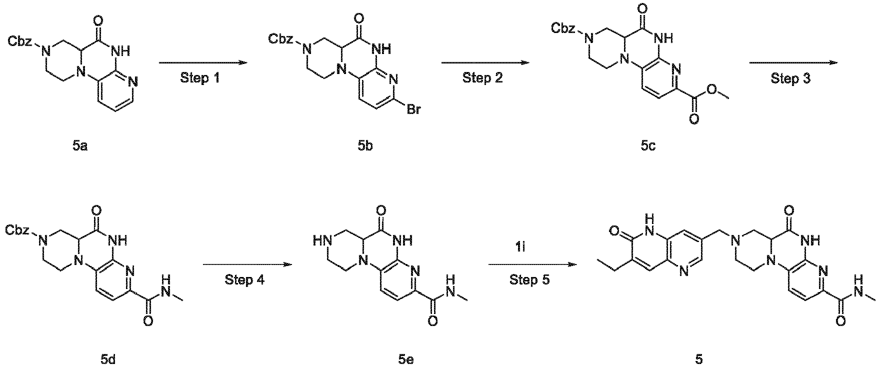

### Step 1

### Benzyl (±)-8-bromo-5-oxo-1,2,4,4a,5,6-hexahydro-3H-pyrazino[1,2-a]pyrido[2,3-e]pyrazine-3-carboxylate 5b

Compound benzyl (±)-5-oxo-1,2,4,4*a*,5,6-hexahydro-3*H*-pyrazino[1,2-*a*]pyrido[2,3-*e*]pyrazine-3-carboxyl ate **5a** (566 mg, 1.67 mmol, prepared by the method disclosed in Example 7 on page 6 of the specification in the patent application "US4138564A") was dissolved in dichloromethane (20 mL), and *N*-bromosuccinimide (297.7 mg, 1.67 mmol) was added in batches in an ice bath. After the addition was completed, the mixture was reacted for 2 h while maintaining the temperature. The reaction mixture was concentrated under reduced pressure. The residue was purified by silica gel column chromatography using eluent system A to give the title compound **5b** (343 mg, 35.8% yield).

MS m/z (ESI): 417.2 [M+1].

### Step 2

### (±)-3-Benzyl-8-methyl-5-oxo-1,2,4,4a,5,6-hexahydro-3H-pyrazino[1,2-a]pyrido[2,3-e]p yrazine-3,8-dicarboxylate 5c

Compound **5b** (335.00 mg, 802.86 µmol) was dissolved in methanol (5 mL) and *N,N*-dimethylformamide (5 mL), and [1,1'-bis(diphenylphosphino)ferrocene]palladium(II) dichloride dichloromethane complex (327.55 mg, 401.40 µmol) and triethylamine (812.41 mg, 8.02 mmol) were added. The mixture was purged 3 times with carbon monoxide and stirred at 70 °C for 14 h. The reaction mixture was cooled to room temperature and filtered, and the filtrate was concentrated under reduced pressure. The residue was purified by high performance liquid chromatography (Waters-2545, column: SharpSil-T C18, 30 mm × 150 mm, 5 µm; mobile phase: aqueous phase (10 mmol/L ammonium bicarbonate) and acetonitrile; gradient ratio: acetonitrile 35%-45%; flow rate: 30 mL/min) to give the title compound **5c** (20 mg, 6.2% yield).

MS m/z (ESI): 397.2 [M+1].

### Step 3

### Benzyl (±)-8-(methylaminocarbonyl)-5-oxo-1,2,4,4a,5,6-hexahydro-3H-pyrazino[1,2-a]pyrido[ 2,3-e]pyrazine-3-carboxylate 5d

The crude compound **5c** (20 mg, 48.7 µmol) was dissolved in ethanol (5 mL), a solution of methylamine in ethanol (5 mL, 1 M) was added, and the mixture was stirred for 14 h. The reaction mixture was concentrated under reduced pressure to give crude title compound **5d** (18 mg, 93.4% yield), which was directly used in the next reaction without purification.

MS m/z (ESI): 396.2 [M+1].

### Step 4

### (±)-N-Methyl-5-oxo-2,3,4,4a,5,6-hexahydro-1H-pyrazino[1,2-a]pyrido[2,3-e]pyrazine-8-carboxamide 5e

The crude compound **5d** (20 mg, 50.58 µmol) was dissolved in a solution of hydrobromic acid in acetic acid (2 mL, 30%), and the mixture was stirred for 1.5 h. The reaction mixture was concentrated under reduced pressure to give crude title compound **5e** (10 mg, 75% yield), which was directly used in the next reaction without purification.

MS m/z (ESI): 262.2 [M+1].

### Step 5

### (±)-3-((7-Ethyl-6-oxo-5,6-dihydro-1,5-naphthyridin-3)-yl)methyl)-N-methyl-5-oxo-2,3,4

,4*a*,5,6-hexahydro-1*H*-pyrazino[1,2-*a*]pyrido[2,3-*e*]pyrazine-8-carboxamide **5** Compound **5e** (11 mg, 42.1 µmοl), compound **1i** (10 mg, 44.9 µmol), *N,N*-diisopropylethylamine (29 mg, 224.3 µmol), and sodium iodide (2 mg, 13 µmοl) were dissolved in acetonitrile (3 mL), and the mixture was reacted at 80 °C for 5 h. The reaction mixture was concentrated under reduced pressure. The crude product was purified by high performance liquid chromatography (Waters-2545, column: SharpSil-T C18, 30 mm × 150 mm, 5 µm; mobile phase: aqueous phase (10 mmol/L ammonium bicarbonate) and acetonitrile; gradient ratio: acetonitrile 35%-45%; flow rate: 30 mL/min) to give the title compound **5** (3 mg, 14.9% yield).

MS m/z (ESI): 448.2 [M+1].

1H NMR (500 MHz, CD₃OD): δ 8.56 (d, 1H), 7.84 (s, 1H), 7.77 (d, 1H), 7.71 (d, 1H), 7.24 (d, 1H), 5.34 (t, 2H), 3.89-3.79 (m, 3H), 2.93 (s, 1H), 2.67 (q, 2H), 2.41-2.26 (m, 2H), 2.19 (t, 2H), 1.64-1.57 (m, 2H), 0.90 (t, 3H).

### Example 6

### (±)-3-((7-Ethyl-6-oxo-5,6-dihydro-1,5-naphthyridin-3-yl)methyl)-N-methyl-2,3,4,4a,5,6 -hexahydro-1H-pyrazino[1,2-a]pyrido[2,3-e]pyrazine-8-carboxamide 6

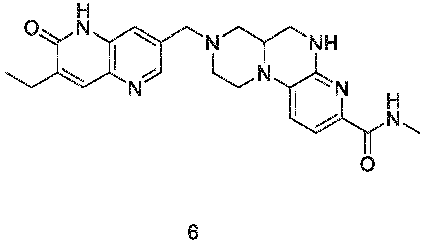

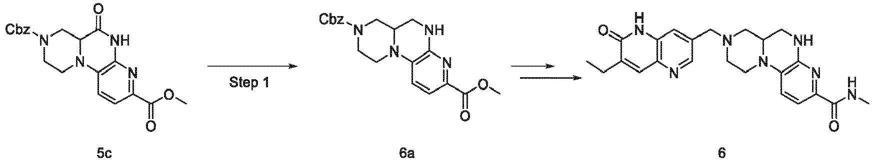

### Step 1

### (±)-3-Benzyl-8-methyl-1,2,4,4a,5,6-hexahydro-3H-pyrazino[1,2-a]pyrido[2,3-e]pyrazin e-3,8-dicarboxylate 6a

Compound **5c** (7 mg, 17.65 µmοl) was dissolved in tetrahydrofuran (1 mL), a solution of borane in tetrahydrofuran (0.2 mL, 1 M) was added, and the mixture was heated to 40 °C and reacted for 1 h. After the reaction was completed, methanol was added, and the mixture was stirred for 1 h. The reaction mixture was concentrated under reduced pressure. The residue was purified by thin-layer chromatography using developing solvent system A to give the title compound **6a** (4 mg, 59.2% yield).

MS m/z (ESI): 383.2 [M+1].

Subsequently, the title compound **6** (1 mg, 21.6% yield) was obtained by following the synthetic route of Example 5 with the starting material compound **5c** in step 3 replaced by compound **6a.**

MS m/z (ESI): 434.2 [M+1].

1H NMR (500 MHz, CDCl₃): δ 9.38 (s, 1H), 8.51 (s, 1H), 7.85 (s, 1H), 7.63 (s, 1H), 7.54-7.45 (m, 2H), 7.07 (d, 1H), 6.86 (d, 1H), 4.72 (s, 1H), 3.77-3.59 (m, 3H), 3.44-3.33 (m, 2H), 3.28-3.19 (m, 1H), 3.07-2.87 (m, 4H), 2.85-2.65 (m, 3H), 2.25 (td, 2H), 0.90 (dt, 3H).

### Example 7

### (±)-3-((7-Ethyl-6-oxo-5,6-dihydro-1,5-naphthyridin-3-yl)methyl)-N,6-dirnethyl-2,3,4,4a ,5,6-hexahydro-1H-pyrazino[1,2-a]pyrido[2,3-e]pyrazine-8-carboxamide 7

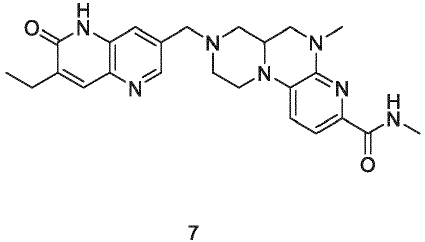

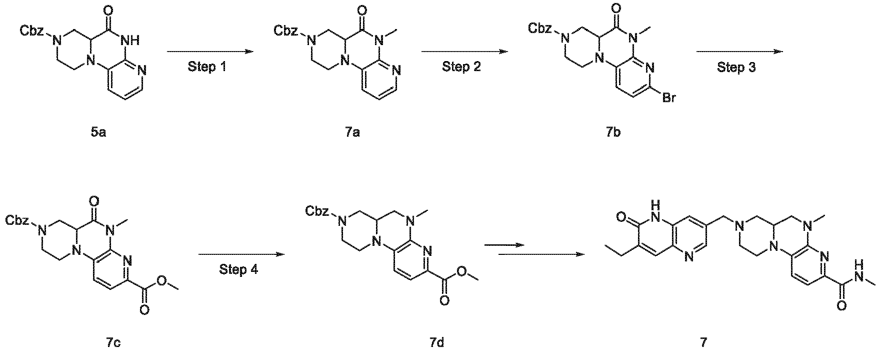

### Step 1

### Benzyl (±)-6-methyl-5-oxo-1,2,4,4a,5,6-hexahydro-3H-pyrazino[1,2-a]pyrido[2,3-e]pyrazine-3 -carboxylate 7a

Compound **5a** (1.50 g, 4.43 mmol) was dissolved in tetrahydrofuran (10 mL), sodium hydride (265.96 mg, 6.64 mmol, 60% purity) was added in an ice bath, and then the mixture was allowed to return to room temperature and stirred for 0.5 h. Iodomethane (1.88 g, 13.24 mmol) was added in an ice bath, and the mixture was stirred for 5 h. The reaction mixture was concentrated under reduced pressure. The residue was purified by silica gel column chromatography using eluent system A to give the title compound **7a** (940 mg, 60.2% yield).

MS m/z (ESI): 353.2 [M+1].

### Step 2

### Benzyl (±)-8-bromo-6-methyl-5-oxo-1,2,4,4a,5,6-hexahydro-3H-pyrazino[1,2-a]pyrido[2,3-e]p yrazine-3-carboxylate 7b

Compound **7a** (620 mg, 1.75 mmol) was dissolved in dichloromethane (10 mL), and *N*-bromosuccinimide (313.15 mg, 1.75 mmol) was added in batches in an ice bath. After the addition was completed, the mixture was reacted for 1 h while maintaining the temperature. The reaction mixture was concentrated under reduced pressure. The residue was purified by silica gel column chromatography using eluent system B to give the title compound **7b** (660 mg, 86.9% yield).

MS m/z (ESI): 431.2 [M+1].

### Step 3

### (±)-3-Benzyl-8-methyl-6-methyl-5-oxo-1,2,4,4a,5,6-hexahydro-3H-pyrazino[1,2-a]pyri do[2,3-e]pyrazine-3,8-dicarboxylate 7c

Compound **7b** (220 mg, 510.1 µmοl) was dissolved in methanol (5 mL) and *N,N*-dimethylformamide (5 mL), and [1,1'-bis(diphenylphosphino)ferrocene]palladium(II) dichloride dichloromethane complex (208.1 mg, 255 µmοl) and triethylamine (516.1 mg, 5.1 mmol) were added. The mixture was purged 3 times with carbon monoxide and stirred at 70 °C for 14 h. The reaction mixture was concentrated under reduced pressure. The residue was purified by silica gel column chromatography using eluent system B to give the title compound **7c** (180 mg, 86.1% yield).

MS m/z (ESI): 411.2 [M+1].

### Step 4

### (±)-3-Benzyl-8-methyl-6-methyl-1,2,4,4a,5,6-hexahydro-3H-pyrazino[1,2-a]pyrido[2,3-e]pyrazine-3,8-dicarboxylate 7d

Compound **7c** (80 mg, 194.92 µmοl) was dissolved in tetrahydrofuran (4 mL), a solution of borane in tetrahydrofuran (2 mL, 1 M) was added, and the mixture was heated to 50 °C and reacted for 1 h. After the reaction was completed, methanol was added, and the mixture was stirred for 1 h. The reaction mixture was concentrated under reduced pressure. The residue was purified by silica gel column chromatography using eluent system B to give the title compound **7d** (61 mg, 78.9% yield).

MS m/z (ESI): 397.2 [M+1].

Subsequently, the title compound 7 (5.4 mg, 8% yield) was obtained by following the synthetic route of Example 5 with the starting material compound **5c** in step 3 replaced by compound **7d.**

MS m/z (ESI): 448.2 [M+1].

1H NMR (500 MHz, CDCl₃): δ 10.58 (s, 1H), 8.52 (s, 1H), 7.87 (s, 1H), 7.73 (d, 1H), 7.66 (s, 1H), 7.48 (d, 1H), 6.78 (d, 1H), 3.80-3.65 (m, 2H), 3.62 (d, 1H), 3.40-3.22 (m, 3H), 3.10 (s, 3H), 3.03 (d, 3H), 2.96-2.82 (m, 2H), 2.74 (q, 2H), 2.40 (t, 1H), 2.09-1.90 (d, 2H), 1.37-1.31 (m, 3H).

### Example 8

### (R)-3-((7-Ethyl-6-oxo-5,6-dihydro-1,5-naphthyridin-3-yl)methyl)-N-methyl-1,2,3,4,4a,5 -hexahydro-7H-pyrazino[2,1-c]pyrido[3,2-e][1,4]oxazepine-9-carboxamide 8

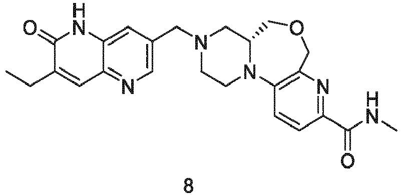

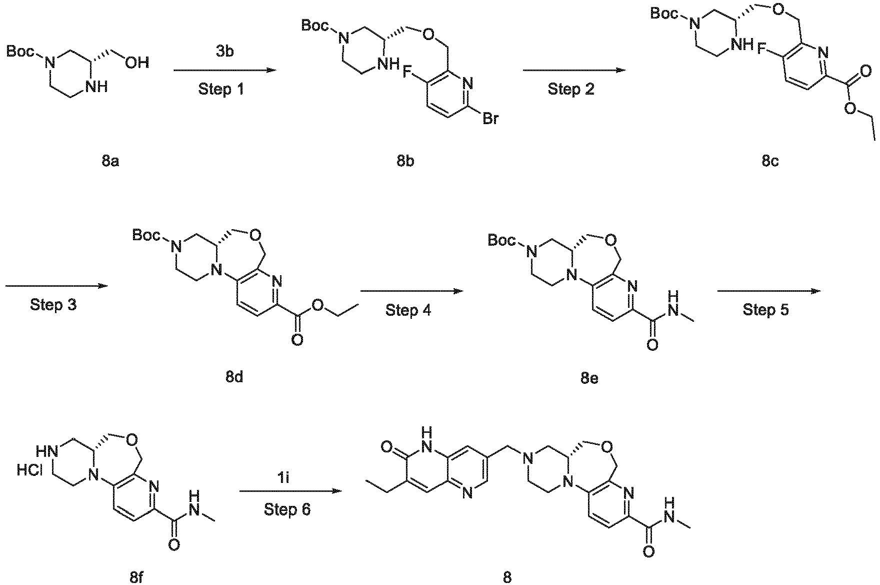

### Step 1

### tert-Butyl (R)-3-(((6-bromo-3-fluoropyridin-2-yl)methoxy)methyl)piperazine-1-carboxylate 8b

Compound **3b** (2.5 g, 9.29 mmol) and compound *tert*-butyl (*R*)-3-(hydroxymethyl)piperazine-1-carboxylate **8a** (2.25 g, 10.40 mmol, Shanghai Hanhong Scientific Co., Ltd.) were dissolved in tetrahydrofuran (30 mL), sodium hydride (812.5 mg, 21.20 mmol, 60% purity) was added in an ice bath, and the mixture was stirred for 2 h. The reaction mixture was concentrated under reduced pressure. The residue was purified by silica gel column chromatography using eluent system A to give the title compound **8b** (3 g, 79.8% yield).

MS m/z (ESI): 404.1 [M+1].

### Step 2

### tert-Butyl (R)-3-(((6-(ethoxycarbonyl)-3-fluoropyridin-2-yl)methoxy)methyl)piperazine-1-carboxy late 8c

Compound **8b** (2 g, 4.94 mmol) was dissolved in a mixed solvent of *N*,*N*-dimethylformamide (20 mL) and ethanol (10 mL), bis(triphenylphosphine)palladium(II) dichloride (0.52 g, 740.8 µmοl) and *N,N*-diisopropylethylamine (1.52 g, 15 mmol) were added, and the mixture was stirred in a carbon monoxide atmosphere at 100 °C for 14 h. The reaction mixture was cooled, diluted by adding ethyl acetate (100 mL), and washed sequentially with water and a saturated sodium chloride solution. The organic phase was collected, dried over anhydrous sodium sulfate, and filtered to remove the desiccant, and then the filtrate was concentrated under reduced pressure. The residue was purified by silica gel column chromatography using eluent system B to give the title compound **8c** (1.5 g, 76.2% yield).

MS m/z (ESI): 398.2 [M+1].

### Step 3

### 3-(tert-Butyl) 9-ethyl-(R)-1,2,4a,5-tetrahydro-7H-pyrazino[2,1-c]pyrido[3,2-e][1,4]oxazepine-3,9(4H) -dicarboxylate 8d

Compound **8c** (4 g, 10.06 mmol) was dissolved in *N*,*N*-dimethylacetamide (20 mL), *N*,*N*-diisopropylethylamine (4 g, 30.9 mmol) was added, and the mixture was reacted under microwave at 140 °C for 6 h. The reaction mixture was cooled and concentrated under reduced pressure. The residue was purified by silica gel column chromatography using eluent system A to give the title compound **8d** (2.3 g, 60% yield).

MS m/z (ESI): 378.2 [M+1].

### Step 4

### tert-Butyl (R)-9-(methylaminocarbonyl)-1,2,4a,5-tetrahydro-7H-pyrazino[2,1-c]pyrido[3,2-e][1,4] oxazepine-3(4H)-carboxylate 8e

Compound **8d** (600 mg, 1.58 mmol) was dissolved in a solution of methylamine in ethanol (5 mL, 1 M), and the mixture was stirred for 14 h. The reaction mixture was concentrated under reduced pressure to give crude title compound **8e** (570 mg, 98% yield), which was directly used in the next reaction without purification.

MS m/z (ESI): 363.2 [M+1].

### Step 5

### (R)-N-Methyl-1,2,3,4,4a,5-hexahydro-7H-pyrazino[2,1-c]pyrido[3,2-e][1,4]oxazepine-9 -carboxamide hydrochloride 8f

The crude compound **8e** (140 mg, 386.2 µmol) was dissolved in dichloromethane (3 mL), a solution of hydrochloric acid in dioxane (1 mL, 4 M) was added, and the mixture was stirred for 2 h. The reaction mixture was concentrated under reduced pressure to give crude title compound **8f** (110 mg, 95% yield), which was directly used in the next reaction without purification.

MS m/z (ESI): 263.2 [M+1].

### Step 6

### (R)-3-((7-Ethyl-6-oxo-5,6-dihydro-1,5-naphthyridin-3-yl)methyl)-N-methyl-1,2,3,4,4a,5 -hexahydro-7H-pyrazino[2,1-c]pyrido[3,2-e][1,4]oxazepine-9-carboxamide 8

The crude compound **8f** (570 mg, 1.9 mol), compound **1i** (430 mg, 1.93 mol), and *N,N*-diisopropylethylamine (1.5 g, 11.6 mmol) were dissolved in acetonitrile (30 mL), sodium iodide (30 mg, 200 µmοl) was added, and the mixture was reacted at 80 °C for 5 h. The reaction mixture was concentrated under reduced pressure. The crude product was purified by high performance liquid chromatography (Waters-2545, column: SharpSil-T C18, 30 mm × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compound **8** (5.4 mg, 8% yield).

MS m/z (ESI): 449.2 [M+1].

1H NMR (500 MHz, CD₃OD): δ 8.51 (d, 1H), 7.93 (d, 1H), 7.86 (s, 1H), 7.79 (d, 1H), 7.50 (d, 1H), 4.99 (t, 2H), 4.85 (d, 1H), 4.08 (dd, 2H), 3.87 (dd, 1H), 3.81-3.71 (m, 2H), 3.46 (ddd, 2H), 2.95 (s, 3H), 2.86- 2.78 (m, 1H), 2.75-2.61 (m, 3H), 2.57 (dd, 1H), 1.31 (t, 3H).

### Example 9

### (R)-3-((5-Fluoro-2-methyl-3-oxo-3,4-dihydroquinoxalin-6-yl)methyl)-N-methyl-1,2,3,4, 4a,5-hexahydro-7H-pyrazino[2,1-c]pyrido[3,2-e][1,4]oxazepine-9-carboxamide 9

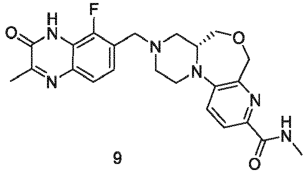

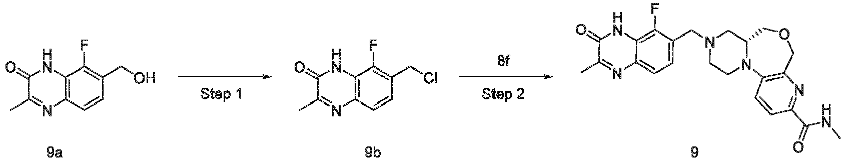

### Step 1

### 7-(Chloromethyl)-8-fluoro-3-methylquinoxalin-2(1H)-one 9b

Compound **9a** (100 mg, 480 µmol, prepared by the method disclosed in Intermediate 17 on page 35 of the specification of the patent application "WO2021260093 A1") was dissolved in dichloromethane (4 mL), phosphorus oxychloride (3 mL) was added, and the mixture was stirred for 14 h. The reaction mixture was concentrated under reduced pressure, ice water was added, and a solid precipitated. The mixture was filtered, and the filter cake was washed with water and dried to give crude title compound **9b** (90 mg), which was directly used in the next reaction without purification.

MS m/z (ESI): 227.2 [M+1].

### Step 2

### (R)-3-((5-Fluoro-2-methyl-3-oxo-3,4-dihydroquinoxalin-6-yl)methyl)-N-methyl-1,2,3,4, 4a,5-hexahydro-7H-pyrazino[2,1-c]pyrido[3,2-e][1,4]oxazepine-9-carboxamide 9

The crude compound **9b** (136 mg, 601 µmol), compound **8f** (255 mg, 601 µmol), *N,N*-diisopropylethylamine (233 mg, 1.8 mmol), and sodium iodide (18 mg, 125.6 µmοl) were dissolved in acetonitrile (3 mL), and the mixture was reacted at 80 °C for 3 h. The reaction mixture was concentrated under reduced pressure. The crude product was purified by high performance liquid chromatography (Waters-2545, column: YMC Triart-Exrs, Prep 30 mm × 150 mm, 5 µm, C18; mobile phase: aqueous phase (10 mmol/L ammonium bicarbonate) and acetonitrile; gradient ratio: acetonitrile 35%-45%; flow rate: 30 mL/min) to give the title compound **9** (40 mg, 14.6% yield).

MS m/z (ESI): 453.2 [M+1].

¹H NMR (500 MHz, CD₃OD): δ 7.93 (d, 1H), 7.57 (d, 1H), 7.48 (d, 1H), 7.40 (t, 1H), 4.97 (d, 1H), 4.91 (s, 1H), 4.01 (dd, 1H), 3.85 (dd, 1H), 3.80 (s, 2H), 3.41 (td, 3H), 2.95 (s, 3H), 2.83 (dq, 1H), 2.73 (dd, 1H), 2.60 (ddd, 2H), 2.53 (s, 3H).

### Example 10

### (R)-3-((5-Fluoro-2-methyl-3-oxo-3,4-dihydroquinoxalin-6-yl)methyl)-N-methyl-1,2,3,4, 4a,5-hexahydropyrazino[1,2-d]pyrido[2,3-b][1,4]oxazine-8-carboxamide 10

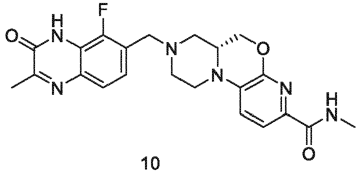

The title compound **10** (84 mg, 30.5% yield) was obtained by following the synthetic route of Example 2 with the starting material compound **1i** in step 6 replaced by compound **9b.**

MS m/z (ESI): 439.2 [M+1].

¹H NMR (500 MHz, CD₃OD): δ 7.63 (d, 1H), 7.57 (d, 1H), 7.41 (t, 1H), 7.25 (d, 1H), 4.42 (dd, 1H), 4.13 (t, 1H), 3.80 (d, 3H), 3.29 (s, 3H), 3.08 (d, 1H), 3.00 (d, 1H), 2.94-2.85 (m, 4H), 2.53 (s, 3H).

### Biological Evaluation

The present disclosure is further described and explained below with reference to test examples. However, these test examples are not intended to limit the scope of the present disclosure.

### Test Example 1. Cell Proliferation Assay

The following method evaluates the inhibitory effect of the compounds of the present disclosure on the proliferation of DLD1, DLD1^{BRCA2-/-}, and MDA-MB-436 cells via IC₅₀ by measuring the intracellular ATP content. The experimental method was briefly described as follows:

### I. Experimental materials and instruments

1. DLD1, human colon cancer cells (Cobioer, Nanjing, CBP60037)
2. DLD1^{BRCA2-/-}, human BRCA2 gene knock-out colon cancer cells (Creative biogene, CSC-RT0015)
3. MDA-MB-436, human breast cancer cells (ATCC, HTB-130)
4. Fetal bovine serum (GIBCO, 10091-148)
5. CellTite-Glo reagent (Promega, G7573)
6. 96-well cell culture plate (corning, 3903)
7. Pancreatin (invitrogen, 25200-072)
8. Microplate reader (BMG, PHERAsta)
9. Cell counter (Countstar, Shanghai, IC1000)

### II. Experimental procedures

DLD1 cells were cultured in an RPMI-1640 medium containing 10% FBS, and passaged 2-3 times a week in a passage ratio of 1:6 or 1:8. During passage, the cells were digested by pancreatin, transferred to a centrifuge tube, and centrifuged for 3 min at 1200 rpm. The supernatant was discarded, and a fresh medium was added to resuspend the cells. To a 96-well cell culture plate, 180 µL of the cell suspension was added at a density of 2.78 × 10³ cells/mL. To peripheral wells of the 96-well plate, only 180 µL of complete medium was added.

DLD1^{BRCA2-/-} cells were cultured in an RPMI-1640 medium containing 10% FBS, and passaged 2-3 times a week in a passage ratio of 1:6 or 1:8. During passage, the cells were digested by pancreatin, transferred to a centrifuge tube, and centrifuged for 3 min at 1200 rpm. The supernatant was discarded, and a fresh medium was added to resuspend the cells. To a 96-well cell culture plate, 180 µL of the cell suspension was added at a density of 8.34 × 10³ cells/mL. To peripheral wells of the 96-well plate, only 180 µL of complete medium was added.

MDA-MB-436 cells were cultured in a Leibovitz's L-15 medium containing 10% FBS, 10 µg/mL insulin, and 16 µg/mL glutathione, and passaged 2-3 times a week in a passage ratio of 1:3 or 1:5. During passage, the cells were digested by pancreatin, transferred to a centrifuge tube, and centrifuged for 3 min at 1200 rpm. The supernatant was discarded, and a fresh medium was added to resuspend the cells. To a 96-well cell culture plate, 180 µL of the cell suspension was added at a density of 8.34 × 10³ cells/mL. To peripheral wells of the 96-well plate, only 180 µL of complete medium was added.

The culture plate was incubated in an incubator for 24 h (37 °C, 5% CO₂).

The test samples were diluted to 2 mM in DMSO and serially 3-fold diluted to the 10^{th} concentration. Blank and control wells were set. 5 µL of the serially diluted test compound solutions was added to 95 µL of fresh medium. 20 µL of the medium solution containing the compound described above was added to the plate. The plate was incubated in an incubator (37 °C, 5% CO₂) for 6 days. 90 µL of CellTiter-Glo reagent was added to each well of the 96-well cell culture plate. The plate was let stand for 5-10 min in the dark at room temperature. The chemiluminescence signals were read by a PHERAstar system, and the data were processed by GraphPad software. The signal values are shown in Table 1.

**Table 1. Inhibitory effects of the compounds of the present disclosure on the proliferation of DLD1, DLD1^{BRCA2-/-}, and MDA-MB-436 cells**

| Example No. | DLD1 IC₅₀ (nM) | DLD1^{BRCA2-/-} IC₅₀ (nM) | MDA-MB-436 IC₅₀ (nM) |
|---|---|---|---|
| 1 | >1000 | 7.9 | 5.8 |
| 2 | >250 | 2.5 | 1.0 |
| 3 | >250 | 5.8 | 1.1 |
| 6 | >1000 | - | 41.8 |
| 7 | >1000 | - | 18.7 |
| 8 | >1000 | 7.84 | 1.2 |

Conclusion: the compounds of the present disclosure have relatively good inhibitory effects on the proliferation of DLD1^{BRCA2-/-} and MDA-MB-436 cells.

### Test Example 2. Assay on Binding Activities of Compounds of the Present Disclosure for PARP1 and PARP2

*In vitro* binding activities for PARP1 and PARP2 were tested by the following method.

### I. Materials and instruments

1. PARP1 recombinant protein (Sino Biological, Cat. # 11040-H08B);
2. PARP2 recombinant protein (BPS, Cat. # 80502);
3. Fluorescent probe (made in-house using a compound with Cat. # 1380359-84-1, Shanghai Hengrui);
4. 384-well plate (Corning, 3575);
5. Microplate reader PHERAstar FS (BMG Labtech).

### II. Experimental procedures

To each well of a 384-well plate was added 8 µL of binding buffer. A fluorescent probe was dissolved in dimethyl sulfoxide, the mixture was diluted to the corresponding concentration, and then the fluorescent probe formulated in dimethyl sulfoxide was 20-fold diluted with the binding buffer (50 mM Tris-HCl pH 8.0, 50 mM NaCl, 1 mM MgCl₂, 0.1 mM EDTA, and 0.01% IGEPAL) at 2 µL/well. The test compounds were dissolved in dimethyl sulfoxide and diluted to gradient concentrations as required for the experiment, and the compounds at various concentrations formulated in dimethyl sulfoxide were 20-fold diluted with the binding buffer at 2 µL/well. A PARP1 or PARP2 protein was diluted to the corresponding concentration with the binding buffer, and added to a black 384-well plate at 8 µL/well. The plate was incubated at 25 °C for 40 min after the mixture was uniformly mixed. The signal values were read with the FP program in a microplate reader PHERAstar FS. Data were processed using GraphPad software.

The inhibitory activities of the compounds of the present disclosure on the binding to PARP1 and PARP2 were determined by the above assay. The IC₅₀ values obtained are shown in Table 2.

**Table 2. Inhibitory activities of the compounds of the present disclosure on the binding to PARP1 and PARP2**

| Example No. | PARP1 IC₅₀ (nM) | PARP2 IC₅₀ (nM) |
|---|---|---|
| 2 | 11 | 1779 |
| 8 | 19 | >10000 |

Conclusion: the compounds of the present disclosure have a selective inhibitory effect on PARP1.

## Claims

1. A compound of general formula (IM) or a pharmaceutically acceptable salt thereof: 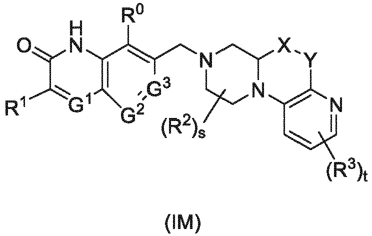 wherein:
X and Y are identical or different and are each independently selected from the group consisting of (CR^{4a}R^{4b})ₘ, NR⁵(CR^{4c}R^{4d})ᵣ, C(O)NR⁵, NR⁵C(O), C(O), and O(CR^{4e}R^{4f})ₙ;
R^{4a}, R^{4b}, R^{4c}, R^{4d}, R^{4e}, and R^{4f} are identical or different and are each independently selected from the group consisting of hydrogen atom, halogen, alkyl, haloalkyl, hydroxyalkyl, cycloalkyl, heterocyclyl, aryl, and heteroaryl;
R⁵ is selected from the group consisting of hydrogen atom, alkyl, haloalkyl, hydroxyalkyl, cycloalkyl, heterocyclyl, aryl, and heteroaryl;
G¹, G², and G³ are identical or different and are each independently CR⁶ or a nitrogen atom;
R⁰, R¹, and R⁶ are identical or different and are each independently selected from the group consisting of hydrogen atom, halogen, alkyl, alkoxy, haloalkyl, haloalkoxy, hydroxyalkyl, cyano, -NR^{7a}R^{7b}, hydroxy, -C(O)R⁸, -C(O)OR⁸, -C(O)NR^{7a}R^{7b}, -S(O)ₚR⁸, cycloalkyl, heterocyclyl, aryl, and heteroaryl, wherein the alkyl, alkoxy, cycloalkyl, heterocyclyl, aryl, and heteroaryl are each independently optionally substituted with one or more substituents selected from the group consisting of halogen, oxo, alkyl, alkoxy, haloalkyl, haloalkoxy, cyano, -NR^{9a}R^{9b}, hydroxy, hydroxyalkyl, cycloalkyl, heterocyclyl, aryl, and heteroaryl;
each R² is identical or different and is independently selected from the group consisting of halogen, alkyl, alkoxy, haloalkyl, haloalkoxy, oxo, cyano, -NR^{7a}R^{7b}, hydroxy, and hydroxyalkyl;
each R³ is identical or different and is independently selected from the group consisting of halogen, alkyl, alkoxy, haloalkyl, haloalkoxy, hydroxyalkyl, cyano, -NR^{7a}R^{7b}, hydroxy, -C(O)R⁸, -C(O)OR⁸, -C(O)NR^{7a}R^{7b}, -S(O)ₚR⁸, cycloalkyl, heterocyclyl, aryl, and heteroaryl, wherein the alkyl, alkoxy, cycloalkyl, heterocyclyl, aryl, and heteroaryl are each independently optionally substituted with one or more substituents selected from the group consisting of halogen, oxo, alkyl, alkoxy, haloalkyl, haloalkoxy, cyano, -NR^{9a}R^{9b}, hydroxy, hydroxyalkyl, cycloalkyl, heterocyclyl, aryl, and heteroaryl;
R^{7a}, R^{7b}, R^{9a}, and R^{9b} are identical or different and are each independently selected from the group consisting of hydrogen atom, alkyl, hydroxyalkyl, cycloalkyl, and heterocyclyl, wherein the alkyl, cycloalkyl, and heterocyclyl are each independently optionally substituted with one or more substituents selected from the group consisting of halogen, alkyl, alkoxy, haloalkyl, and haloalkoxy;
or R^{7a} and R^{7b}, together with the nitrogen atom to which they are attached, form heterocyclyl; R^{9a} and R^{9b}, together with the nitrogen atom to which they are attached, form heterocyclyl; the heterocyclyl formed is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
R⁸ is selected from the group consisting of hydrogen atom, alkyl, hydroxyalkyl, cycloalkyl, and heterocyclyl, wherein the alkyl, cycloalkyl, and heterocyclyl are each independently optionally substituted with one or more substituents selected from the group consisting of halogen, alkyl, alkoxy, haloalkyl, and haloalkoxy;
p is 0, 1, or 2;
m is 0, 1, 2, 3, or 4;
n is 0, 1, 2, 3, or 4;
r is 0, 1, 2, 3, or 4;
s is 0, 1, 2, 3, or 4; and
t is 0, 1, 2, or 3.

2. The compound of general formula (IM) or the pharmaceutically acceptable salt thereof according to claim 1, wherein X is (CR^{4a}R^{4b})ₘ or C(O), wherein R^{4a}, R^{4b}, and m are as defined in claim 1.

3. The compound of general formula (IM) or the pharmaceutically acceptable salt thereof according to claim 1 or 2, wherein Y is O(CR^{4e}R^{4f})ₙ or NR⁵(CR^{4c}R^{4d})ᵣ, wherein R^{4c}, R^{4d}, R^{4e}, R^{4f}, R⁵, n, and r are as defined in claim 1.

4. The compound of general formula (IM) or the pharmaceutically acceptable salt thereof according to any one of claims 1 to 3, being a compound of general formula (II-1) or a pharmaceutically acceptable salt thereof: 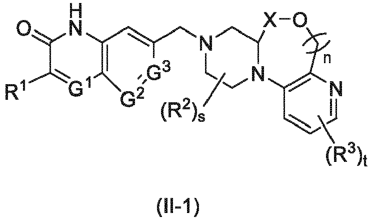 wherein:
X is (CR^{4a}R^{4b})ₘ;
G¹ to G³, R¹ to R³, R^{4a}, R^{4b}, s, t, m, and n are as defined in claim 1.

5. The compound of general formula (IM) or the pharmaceutically acceptable salt thereof according to any one of claims 1 to 4, wherein s is 0.

6. The compound of general formula (IM) or the pharmaceutically acceptable salt thereof according to any one of claims 1 to 5, wherein G¹ is CH, G² is a nitrogen atom, and G³ is CR⁶; or G¹ and G² are both CH, and G³ is a nitrogen atom; or G¹ is a nitrogen atom, G² is CH, and G³ is CR⁶; wherein R⁶ is as defined in claim 1.

7. The compound of general formula (IM) or the pharmaceutically acceptable salt thereof according to any one of claims 1 to 6, wherein R³ is selected from the group consisting of halogen, C₁₋₆ alkyl, C₁₋₆ alkoxy, C₁₋₆ haloalkyl, C₁₋₆ haloalkoxy, C₁₋₆ hydroxyalkyl, cyano, -NR^{7a}R^{7b}, hydroxy, -C(O)R⁸, -C(O)OR⁸, and -C(O)NR^{7a}R^{7b}; preferably, R³ is -C(O)NR^{7a}R^{7b}; wherein R^{7a}, R^{7b}, and R⁸ are as defined in claim 1.

8. The compound of general formula (IM) or the pharmaceutically acceptable salt thereof according to any one of claims 1 to 7, being a compound of general formula (III-1) or general formula (III-I-A) or a pharmaceutically acceptable salt thereof: 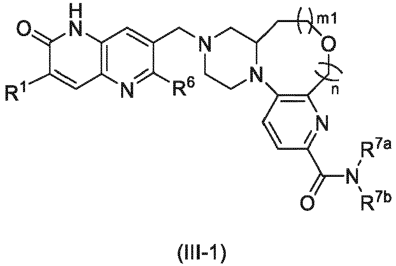 or 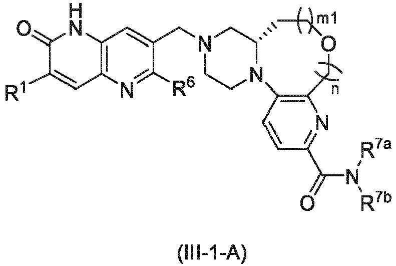 wherein:
m1 is 0 or 1;
R¹, R⁶, R^{7a}, R^{7b}, and n are as defined in claim 1.

9. The compound of general formula (IM) or the pharmaceutically acceptable salt thereof according to any one of claims 1 to 8, wherein R¹ is selected from the group consisting of hydrogen atom, halogen, C₁₋₆ alkyl, C₁₋₆ alkoxy, C₁₋₆ haloalkyl, C₁₋₆ haloalkoxy, and C₁₋₆ hydroxyalkyl.

10. The compound of general formula (IM) or the pharmaceutically acceptable salt thereof according to any one of claims 1 to 9, wherein R⁶ is selected from the group consisting of hydrogen atom, halogen, C₁₋₆ alkyl, C₁₋₆ alkoxy, C₁₋₆ haloalkyl, C₁₋₆ haloalkoxy, and C₁₋₆ hydroxyalkyl.

11. The compound of general formula (IM) or the pharmaceutically acceptable salt thereof according to any one of claims 1 to 10, wherein R^{7a} and R^{7b} are identical or different and are each independently selected from the group consisting of hydrogen atom, C₁₋₆ alkyl, C₁₋₆ hydroxyalkyl, 3- to 8-membered cycloalkyl, and 3- to 8-membered heterocyclyl.

12. The compound of general formula (IM) or the pharmaceutically acceptable salt thereof according to any one of claims 1 to 11, wherein n is 0 or 1.

13. The compound of general formula (IM) or the pharmaceutically acceptable salt thereof according to any one of claims 1 to 12, being selected from the group consisting of any one of the following compounds:

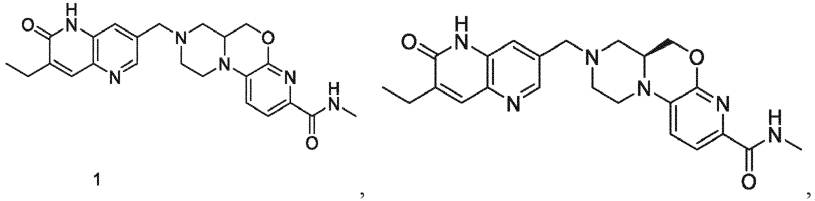

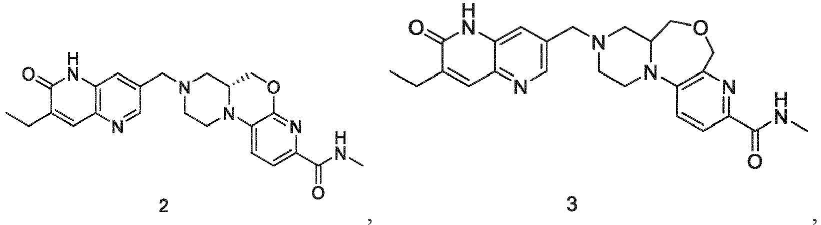

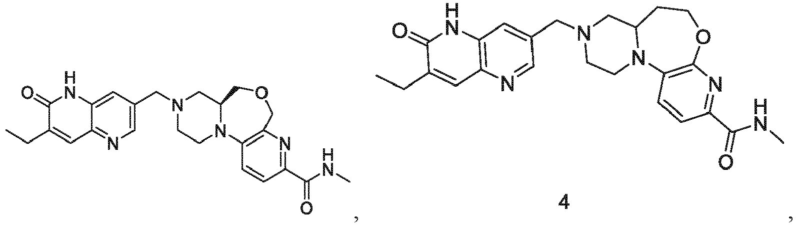

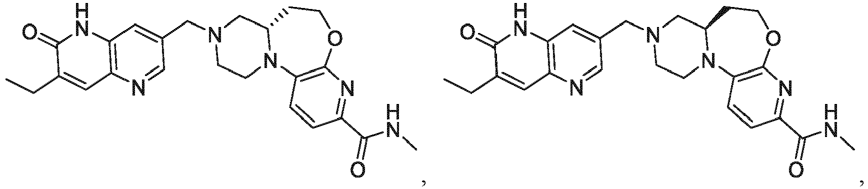

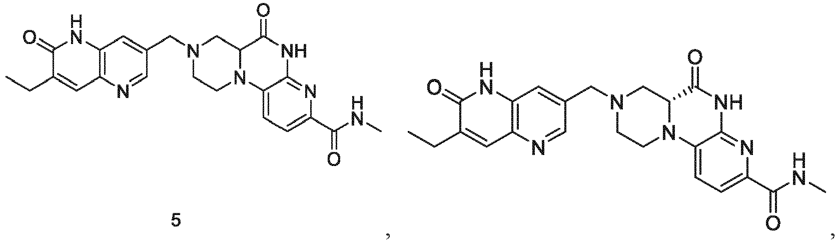

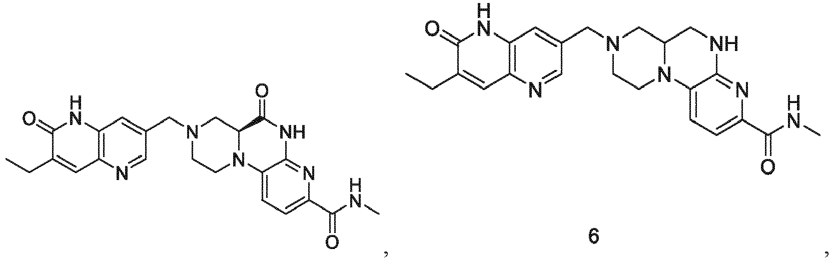

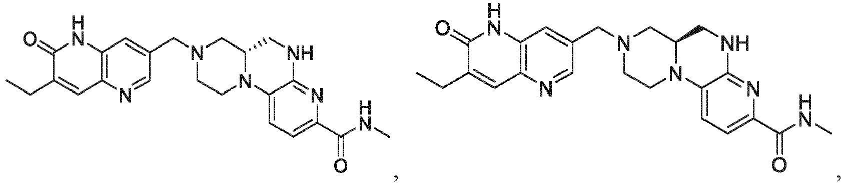

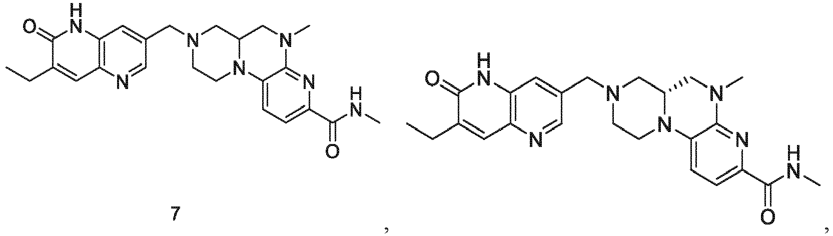

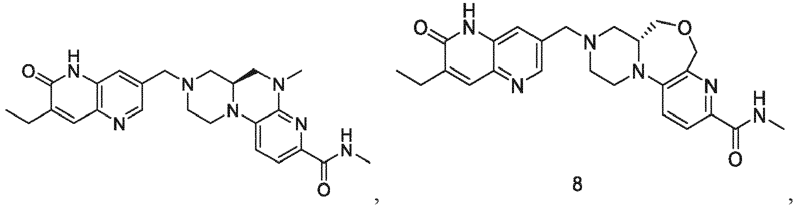

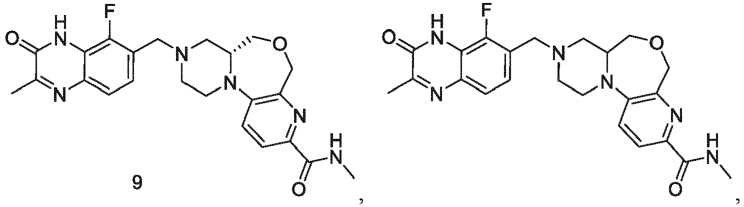

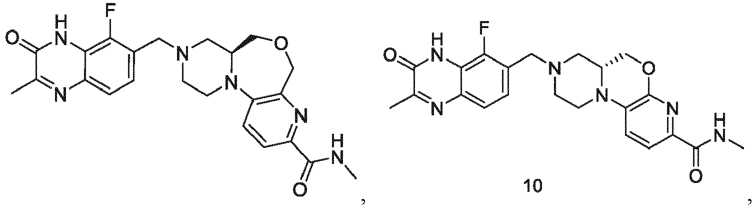

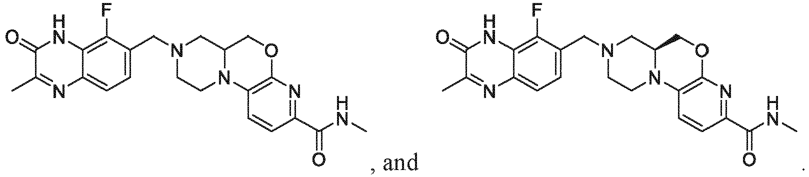

14. A compound of general formula (IMa) or a salt thereof: 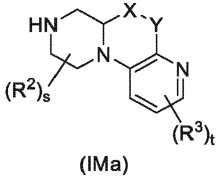 wherein:
t is 1, 2, or 3;
X, Y, R², R³, and s are as defined in claim 1.

15. The compound of general formula (IMa) or the salt thereof according to claim 14, being selected from the group consisting of:

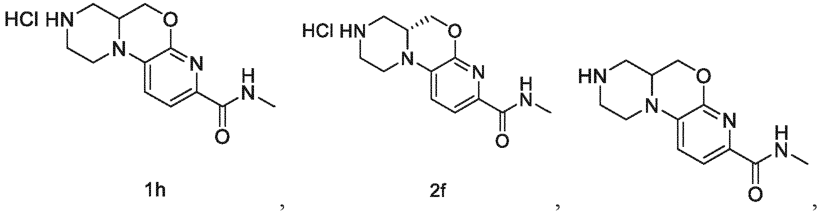

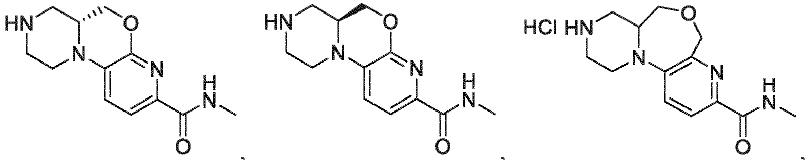

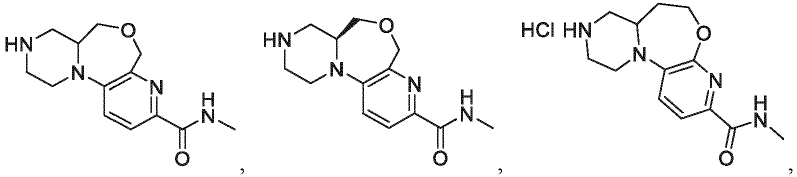

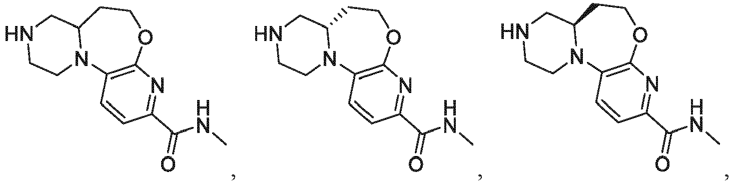

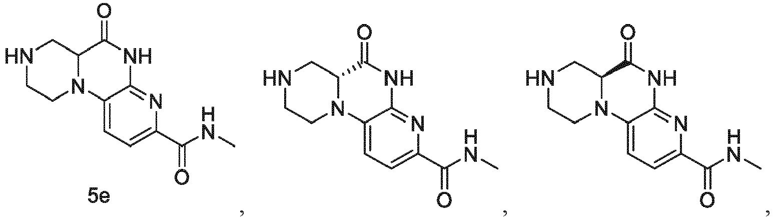

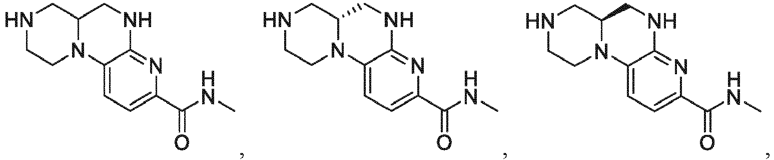

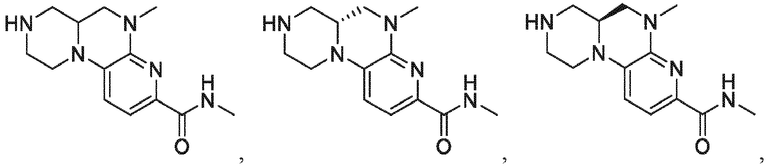

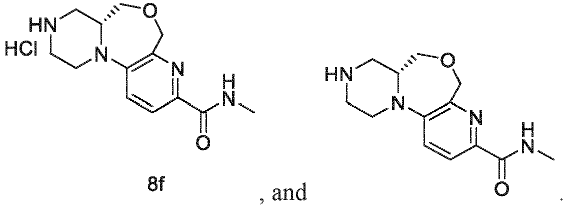

16. A method for preparing the compound of general formula (IM) or the pharmaceutically acceptable salt thereof according to claim 1, wherein the method comprises: 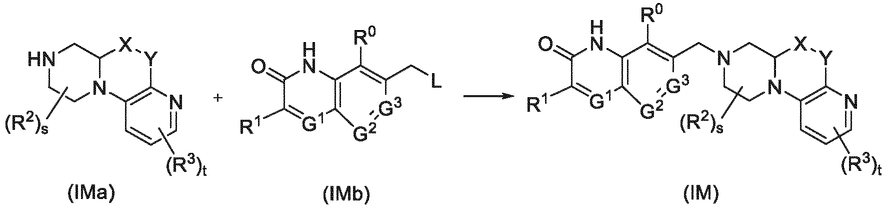
conducting a nucleophilic substitution reaction of a compound of general formula (IMa) or a salt (preferably hydrochloride) thereof with a compound of general formula (IMb) to give the compound of general formula (IM) or the pharmaceutically acceptable salt thereof;
wherein:
L is halogen, preferably a chlorine atom;
X, Y, G¹ to G³, R⁰ to R³, s, and t are as defined in claim 1.

17. A pharmaceutical composition, wherein the pharmaceutical composition comprises the compound of general formula (IM) or the pharmaceutically acceptable salt thereof according to any one of claims 1 to 13, and one or more pharmaceutically acceptable carriers, diluents, or excipients.

18. Use of the compound of general formula (IM) or the pharmaceutically acceptable salt thereof according to any one of claims 1 to 13 or the pharmaceutical composition according to claim 17 in the preparation of a PARP1 inhibitor.

19. Use of the compound of general formula (IM) or the pharmaceutically acceptable salt thereof according to any one of claims 1 to 13 or the pharmaceutical composition according to claim 17 in the preparation of a medicament for treating and/or preventing cancer.

20. The use according to claim 19, wherein the cancer is selected from the group consisting of breast cancer, ovarian cancer, pancreatic cancer, prostate cancer, stomach cancer, colorectal cancer, lung cancer, kidney cancer, liver cancer, cervical cancer, endometrial cancer, myeloma, leukemia, lymphoma, acoustic neuroma, basal cell carcinoma, cholangiocarcinoma, bladder cancer, brain cancer, bronchial cancer, sarcoma, chordoma, choriocarcinoma, craniopharyngioma, cystadenocarcinoma, embryonal carcinoma, hemangioendothelioma, ependymoma, epithelial cancer, esophageal cancer, essential thrombocytosis, Ewing sarcoma, testicular cancer, glioma, heavy chain disease, hemangioblastoma, medullary carcinoma, medulloblastoma, melanoma, meningioma, mesothelioma, neuroblastoma, NUT midline carcinoma, neuroglioma, bone cancer, nasopharyngeal cancer, oral cancer, thyroid cancer, pinealoma, polycythemia vera, retinoblastoma, sebaceous carcinoma, seminoma, skin cancer, squamous cell carcinoma, synovioma, sweat gland carcinoma, Waldenström macroglobulinemia, and Wilms tumor; preferably, the cancer is selected from the group consisting of breast cancer, ovarian cancer, pancreatic cancer, prostate cancer, stomach cancer, colorectal cancer, and lung cancer.
